(19) **Europäisches Patentamt** / **European Patent Office** / **Office européen des brevets**

(11) **EP 3 192 554 A1**

(12) **EUROPEAN PATENT APPLICATION**

(43) Date of publication:
**19.07.2017 Bulletin 2017/29**

(21) Application number: **16151558.0**

(22) Date of filing: **15.01.2016**

(51) Int Cl.:
*A61M 27/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **DEBIOTECH S.A.**
**1004 Lausanne (CH)**

(72) Inventor: **CHAPPEL, Eric**
**1004 Lausanne (CH)**

(74) Representative: **Weihs, Bruno Konrad**
**ANDRE ROLAND SA**
**P.O. Box 5107**
**1002 Lausanne (CH)**

(54) **METHOD OF ADJUSTMENT FOR HYDROCEPHALUS VALVE**

(57)  The present invention describes several methods based on the combination of device setting change with patient postural change to make fast and reliable hydrocephalus valve adjustment. The monitoring and the analysis of the pressure gradient values during these changes allow the tuning of the device, the characterization of a patient physiological characteristic and/or the detection of a failure.

FIG. 1

EP 3 192 554 A1

**Description**

**FIELD OF THE INVENTION**

The present invention relates to a method and system adapted to adjust in vivo an implanted valve for hydrocephalus treatment. The method includes a test method performed in vivo which is adapted to detect a failure of such valve.

**BACKGROUND OF THE INVENTION**

Some people have abnormal accumulation of cerebrospinal fluid (CSF) in the spine and ventricles, or cavities, of the brain. This may cause increased intracranial pressure (ICP) inside the skull. The elevated intracranial pressure may cause compression of the brain, leading to brain damage and other complications. This neurological disease is called hydrocephalus.

For a healthy person, CSF continuously circulates through the ventricles of the brain and the spinal cord. The CSF is continuously drained away into the circulatory system. Hydrocephalus is related to an accumulation of CSF in the brain ventricles. Hydrocephalus is usually caused by a blockage of CSF outflow in the ventricles or in the subarachnoid space over the brain. Even if the outflow passages are blocked, the production of CSF continues, consequently causing an excess of pressure that compresses the nervous tissue and expands the ventricles. Compression of the nervous tissue usually results in irreversible brain damage. If the skull bones are not completely ossified when hydrocephalus occurs, the pressure may also severely enlarge the head. Alternatively, the condition may result from an overproduction of the CSF fluid, from a congenital malformation blocking normal drainage of the fluid, or from complications of head injuries or infections.

Theoretically, hydrocephalus can be successfully treated by placing a drainage tube (shunt) between the brain ventricles and another cavity (often the peritoneum) to eliminate the high internal pressures. Once the hydrocephalus has been diagnosed, a physician performs several tests so as to characterize the cerebrospinal fluid (CSF) dynamics and then he can implant the shunt. It involves the placement of a ventricular catheter into the cerebral ventricles to bypass the flow obstruction and/or drain the excess fluid into other body cavities, from where it can be reabsorbed. Most shunts drain the fluid into the peritoneal cavity (ventriculo-peritoneal shunt, or VP shunt), but alternative sites include the right atrium (ventriculo-atrial shunt), pleural cavity (ventriculo-pleural shunt), and gallbladder. A shunt system can also be placed in the lumbar space of the spine and have the CSF redirected to the peritoneal cavity (LP Shunt).

With the VP shunt, a surgical procedure provides an opening in the skull through which an entry catheter is introduced and passed through the brain tissue into the ventricles. The catheter is fluidly connected to the valve, which is implanted toward the back of the patient's head to control fluid flow. A second exit catheter is also fluidly connected to the valve and leads to the peritoneal cavity.

In a general manner, any information about CSF flow or pressure is fundamental to diagnose and to characterize cerebral disease. In case of abnormal function of the cerebrospinal fluid outflow system (which is reviewed by M. Pollay (Pollay M., The function and structure of the cerebrospinal fluid outflow system, Cerebrospinal Fluid Research 2010, 7:9)), several methods are used to diagnose hydrocephalus. In practice, the constant-rate infusion test is the most frequently used, said method has been proposed by S. Sokolowski (Sokolowski S., Bolus injection test for measurement of cerebrospinal fluid absorption, Journal of the Neurological Sciences 28: 491-504 (1976)). A mathematical modelling of the rise of ICP after injection of a saline solution in the subarachnoid space has been described by Marmarou (Marmarou A. et al., A nonlinear analysis of the cerebrospinal fluid system and intracranial pressure dynamics, J. Neurosurg. 48, 332-344 (1978)). Other mathematical modeling has been described by Clarke at al. (M. Clarke et al., The history of mathematical modeling in hydrocephalus, Neurosurg. Focus 22 (4):E3, 2007) for a review of the history of mathematical modeling in hydrocephalus CSF dynamics) and other refined models are proposed in Smillie A. et al. (Smillie A. et al., A hydro-elastic model of hydrocephalus, Journal of Fluid Mechanics 539: 417-443 (2005)) and Raman K. (Raman K., A stochastic differential equation analysis of cerebrospinal fluid dynamics, Fluids Barriers CNS 8:9 (2011)). A review of the different improvements of this initial model is also proposed by M. Czosnyka et al. (Czosnyka M. et al., Modeling of CSF dynamics: Legacy of Professor Anthony Marmarou, Acta Neurochirurgica Supplementum, Vol. 113, 9 (2012)). All of these models could be used as a theoretical basis for differential diagnosis in hydrocephalus. Nevertheless, these models require infusing a fluid in the cranial cavity so as to increase artificially the fluid in the ventricles which may cause risks for the patient.

Usually, two distinct types of valve are provided to the patient:

- Valve adapted to regulate the fluid flow (a flow regulator).
- Valve adapted to regulate the fluid pressure (a pressure regulator).

The first type of valve allows adjusting the CSF outflow. This valve cannot be regulated after implantation. Thus the physician has to estimate the patient requirement before the surgery. Unfortunately, despite the numerous studies, these valves cannot perfectly be customized before implantation. A cause may be related to the fact that the characterization of the CSF dynamics cannot be 100% accurate; another cause may be that the CSF dynamics changes over time. In many cases, the shunt shall be adjusted after its implantation.

The second type of valve allows adjusting the intracranial pressure (ICP). The valve opens when the fluid pressure reaches a threshold pressure and some of these valves may be tuned in vivo so as to adjust this threshold. The methods used for the adjustment require infusing a fluid in the valve (or upstream the valve). Said adjustment is a critical step and, in most cases, the physician adjusts the setting only depending on the patient feeling. Few shunts comprise an absolute pressure sensor so as to monitor the ICP and the data of the measurement help the caregiver to adjust the setting of the shunt.

Another drawback of these valves is that the valve may open when the patient stands up due to the pressure gradient between the cranial cavity and the peritoneal cavity. Indeed, when the patient is in upright position, the differences in height between the head and the peritoneal cavity (for CP shunt) cause over drainage of CSF. Such over-drainage of CSF could have a negative impact as that would lead to very low ICP (i.e. below -10mmHg) potentially causing tearing of bridging veins or subdural hematomas. Thus, these shunts may comprise an anti-siphon device which stops the flow when the pressure gradient reaches a determined threshold. The physician has to determine said determined threshold which allows closing the valve. Thus, such shunt stops the flow when the patient is in upright position, this induces a progressive growth of the ICP (because when the patient is in upright position, the valve is closed) and it may as well pose an extra risk of the shunt becoming blocked due to a malfunction of the device (constantly closed).

Thus, the shunt has to be regularly monitored so as to re-adjust the setting according to the feedback patient and to check proper functioning of the valve. Furthermore, with the prior art device, it is difficult to detect a failure and it is impossible to determine the type of failure. This induces, if a potential failure is detected, the device is changed and a surgical procedure has to be performed.

The present invention overcomes the drawbacks of the prior art by offering a product and method which improve the patient life by limiting surgical procedures and by offering a valve which is easy to set and which allows detecting several failures and a non-invasive characterization of the CSF dynamics.

## GENERAL DESCRIPTION OF THE INVENTION

A first aspect of the invention is a method of adjustment for in vivo a medical device which comprises the following steps:

- Provide a medical device adapted to regulate a variable (such as a flow rate or a pressure) of the fluid which flows from the cranial cavity to another cavity of the body patient, the medical device comprising:

  ◦ An inlet located in the cranial cavity, in which the fluid enters in the medical device
  ◦ An outlet located in the other cavity, by which the fluid flows out the medical device
  ◦ An adjustable valve in fluid communication with the inlet and the outlet of the medical device, said adjustable valve being adapted to be set by a caregiver in such a way as to adjust the fluid variable,
  ◦ A pressure sensor which senses the differential pressure between the cranial cavity and the other cavity

- Adjust the fluid variable according to a first setting
- Monitor over time the differential pressure,
- Analyze the curve of the monitored differential pressure
- Adjust the fluid variable according to a second setting which taking account the differential pressure analysis

Preferentially, the fluid variable may be the fluid pressure or the flow rate. In one embodiment, the medical device may be an adjustable flow regulator.

To illustrate the method, an example of an adjustable flow regulator is given. Similar flow regulator has been described in the European patent application number EP 09709010.4, EP 09807973.4, EP 11710307.7, EP 14706081.8 and EP 15175963.6 filed by Debiotech SA. The contents of these documents are incorporated by reference in the present account. The principle of such device is based on the elastic distortion of a membrane that goes into contact with a substrate. These flow regulators types comprise a fluid inlet adapted to be connected to a fluid reservoir (for example the cranial cavity) and a fluid outlet adapted to be connected to a delivery location (for example the peritoneal cavity). Said regulators may comprise a substrate and a flexible membrane tightly linked together in predefined linking areas so as to define a cavity there between. Said cavity is fluidly connected to the fluid outlet and to the fluid inlet. The substrate and/or the membrane may comprise a through hole contiguous with said cavity, to define a pathway for a fluid from said fluid inlet to said fluid outlet. Thus, the flexible membrane allows regulating the flow rate (at the outlet) for a predetermined range of a pressure (for example: the transmembrane pressure, i.e. the gradient pressure between the inlet and the outlet). Said flow rate may be substantially constant for said predetermined range of pressure gradient.

In one embodiment, the substrate is at least partially flexible. For example, the substrate may comprise a rigid mesa surrounded by a flexible ring. Thus the mesa may move in a same direction of membrane movement. The bottom of the mesa (for example the mesa side which is not in contact with the cavity) may be operatively coupled (for example attached, glued...) to an element, which allows adjusting the relative position of the mesa. Said adjustment may be performed without contact using adjusting means such as a magnetic rotor. The increase (resp. decrease) of the preload inducing an increase (resp. decrease) of said valve openings and therefore a decrease (resp. an increase) of the flow rate. Thus, the flow rate (at the outlet) can be adjusted by adjusting the relative position of the substrate. In this way, the device can adjust and regulate the flow rate, for a predetermined pressure range. Several pre-settings may be available and the user selects a setting corresponding to a desired flow rate for a pressure range.

Such adjustable valve (i.e. CSF flow regulation as described above) is of a major interest compared to purely passive device because, for a given patient, the production rate of ICP may be not known a priori and it is therefore highly desirable to have a flow regulator having a flow rate that can be adjusted after implantation.

In one embodiment, a sensor may monitor the pressure gradient between the two opposed surfaces of the membrane. In case of hydrocephalus shunt, said pressure gradient may be substantially equal or very close to the pressure gradient between the proximal catheter (i.e. the ICP (intracranial pressure)) and the distal catheter. The sensor may made of strain gages arranged on a surface of or in the flexible membrane.

A second aspect of the invention is a method of detecting a failure of the device. The monitoring of the variation of pressure when the patient moves from horizontal to vertical position may be used as indicator of failure.

Starting from the premise that:

- for a patient in horizontal position, the pressure gradient monitored by the sensor integrated in the flexible membrane is: $\Delta P_h = ICP - PP$; and
- for a patient in vertical position: $\Delta P_v = ICP + HP - PP$

Where PP is the peritoneum pressure.
Where HP is the hydrostatic pressure.
Therefore

$$\Delta P_h - \Delta P_v = HP$$

The value of HP is directly proportional to the head height between the device and the outlet of the distal catheter in the peritoneum (for example from 20 to 40 cm of water corresponding to HP = 20 to 40 mbar). This value can be determined just after implantation to "calibrate" the device, for example by measuring the fluid pressure in upright position and decubitus position.

This mathematical model may be used to detect at least one following failures: total occlusion, partial occlusion and leakage.

A third aspect of the invention is a method for a non-invasive characterization of the CSF dynamics on the logical approach described above.

**LIST OF FIGURES**

Figure 1: Passive flow regulator with drilled membrane and flexible substrate having a mesa.
Figure 2: Adjustable passive flow regulator with drilled membrane with a rigid ball glued on the mesa, the force being applied by a cantilever spring (blade).
Figure 3: Flow rate versus gap for multi-membrane flow regulator.
Figure 4: Mean flow rate in the range 10 to 40 mbar versus the gap.
Figure 5: Inlet and outlet ports on the top.
Figure 6: Pressure gradient through the device for a production rate of 15 ml/h as a function of the gap.
Figure 7: Simulation of the effect of a partial occlusion on the device flow vs pressure characteristics of an auto-regulated shunt.
Figure 8: Pressure drop in the fluidic restriction versus pressure drop in the flow regulation device.
Figure 9: Illustration of the Davson's law.

Figure 10: Pulse amplitude (AMF) versus Intra-Cranial Pressure.

Figure 11: Schematic flow rate through the device after change of the selector position from position i to position i+1 and i-1.

Figure 12: Equivalent electrical model for CSF dynamics analysis of a patient with a shunt.

Figure 13: Evolutions of the relative pressure sensor value and the ICP in case of a sudden increase of the production rate of 2 ml/h at t=0, for a flow regulating valve set at 20 ml/h and for an initial ICP of 10 mbar. Postural changes are described in Table 2.

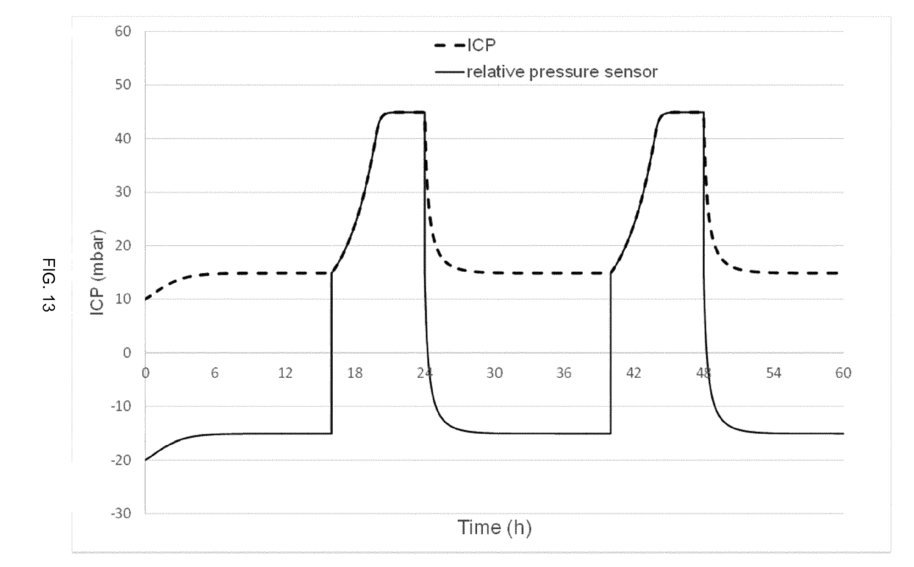

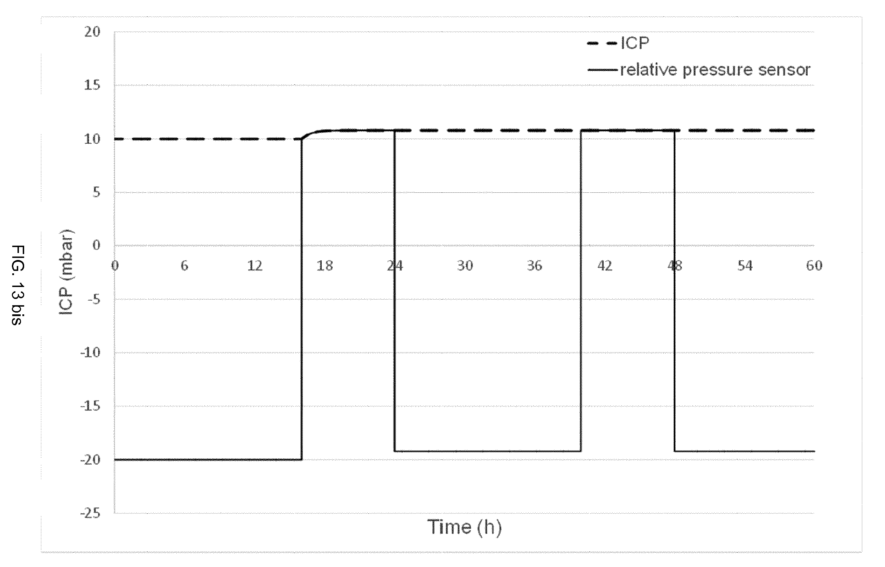

Figure 13 bis: Evolutions of the relative pressure sensor value and the ICP at a nominal production rate of 20 ml/h, for a flow regulating valve set at 20 ml/h and for an initial ICP of 10 mbar. Postural changes are described in Table 2.

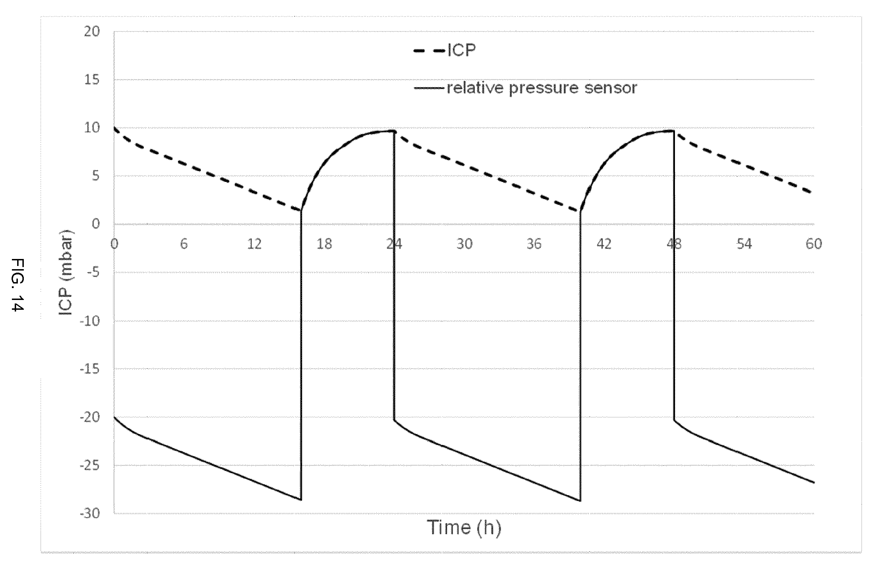

Figure 14: Evolutions of the relative pressure sensor value and the ICP in case of a sudden decrease of the production rate of 2 ml/h at t=0, for a flow regulating valve set at 20 ml/h and for an initial ICP of 10 mbar. Postural changes are described in Table 2.

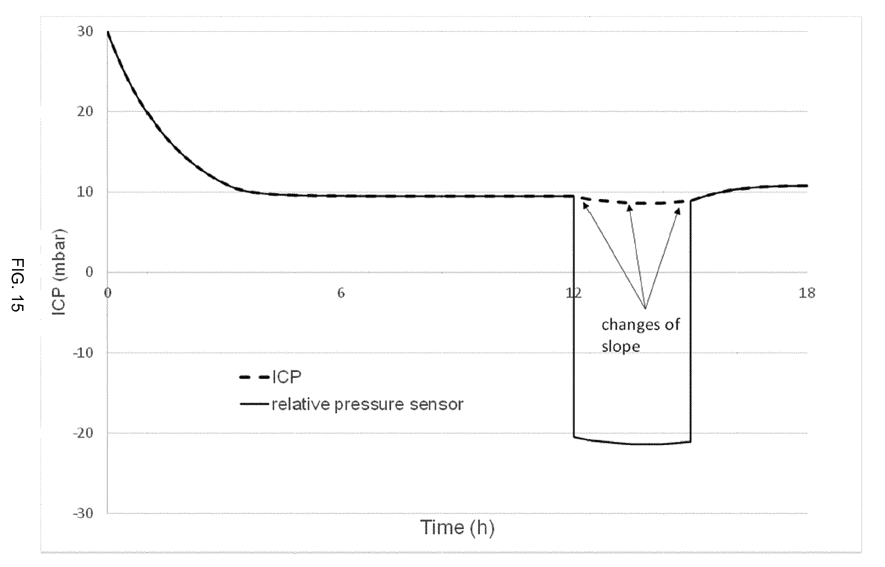

Figure 15: Evolution of the relative pressure sensor value and the ICP according to the tuning procedure described in Table 3.

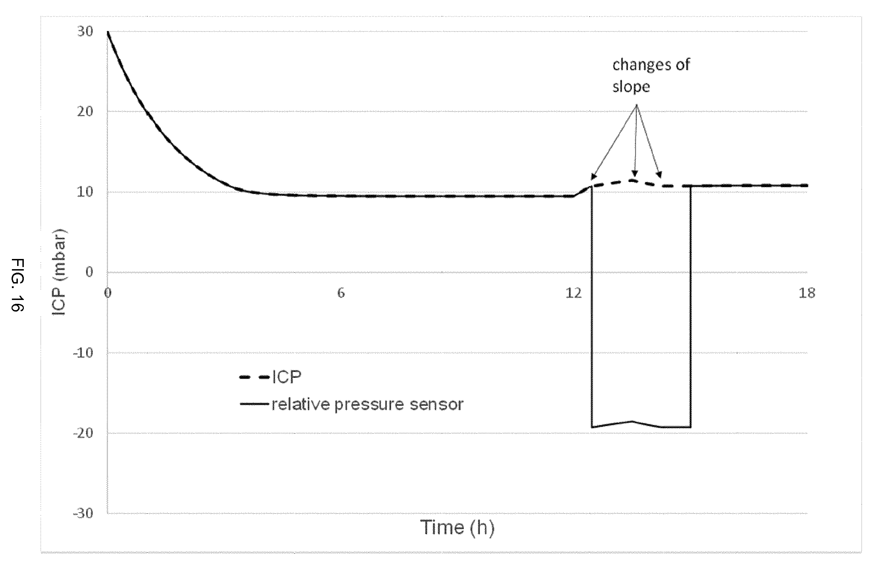

Figure 16: Evolution of the relative pressure sensor value and the ICP according to the tuning procedure described in Table 4.

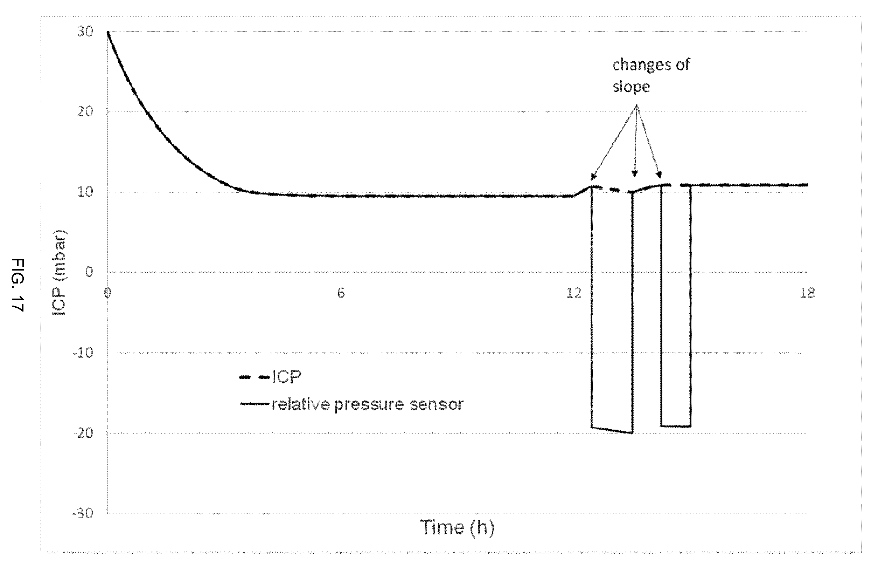

Figure 17: Evolution of the relative pressure sensor value and the ICP according to the tuning procedure described in Table 5.

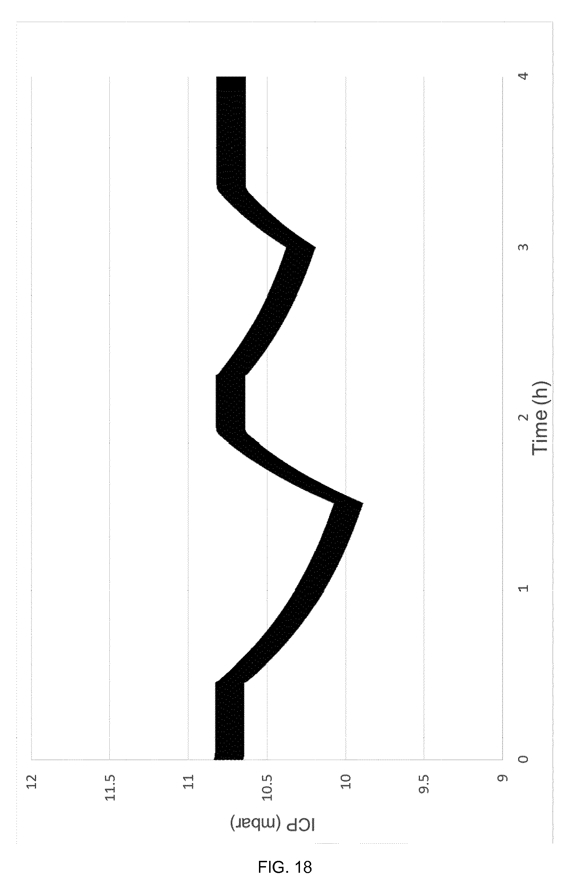

Figure 18: Evolution of the ICP according to the tuning procedure described in Table 5, numerical constants being given in Table 6. The signal during the first 12h of stabilization is not shown.

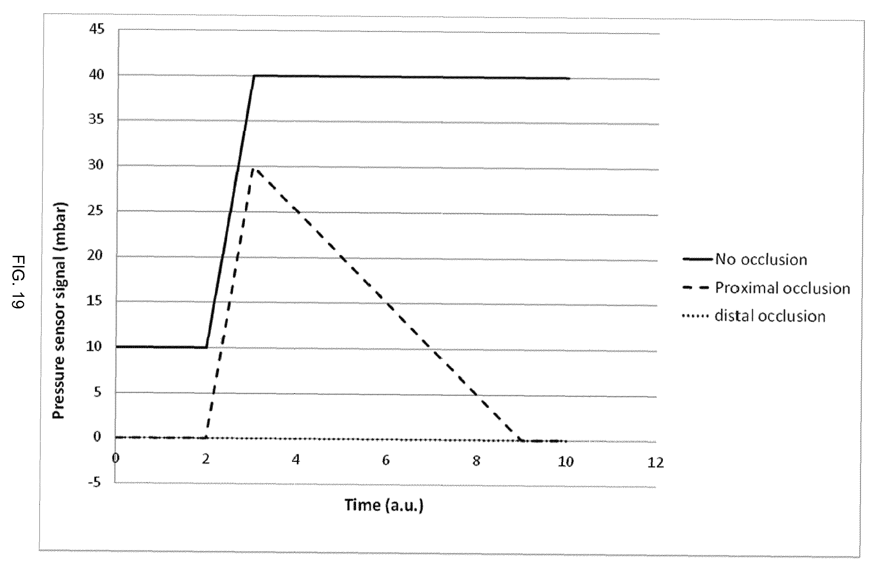

Figure 19: Typical pressure profile in case of postural change (decubitus to upright position) in various conditions of occlusion, for a device exhibiting a large compliance.

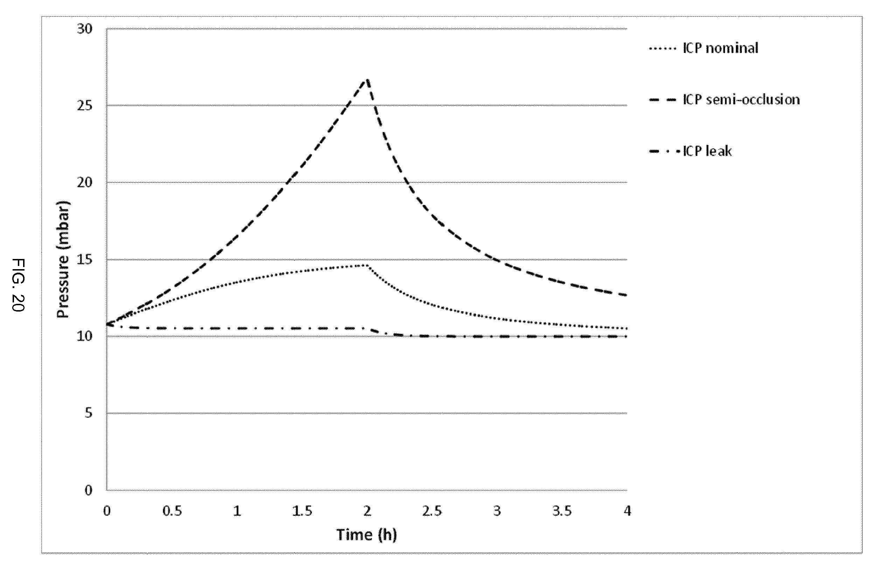

Figure 20: Evolution of the ICP according to the test protocol given in Table 7, using simulation data given in Table 6.

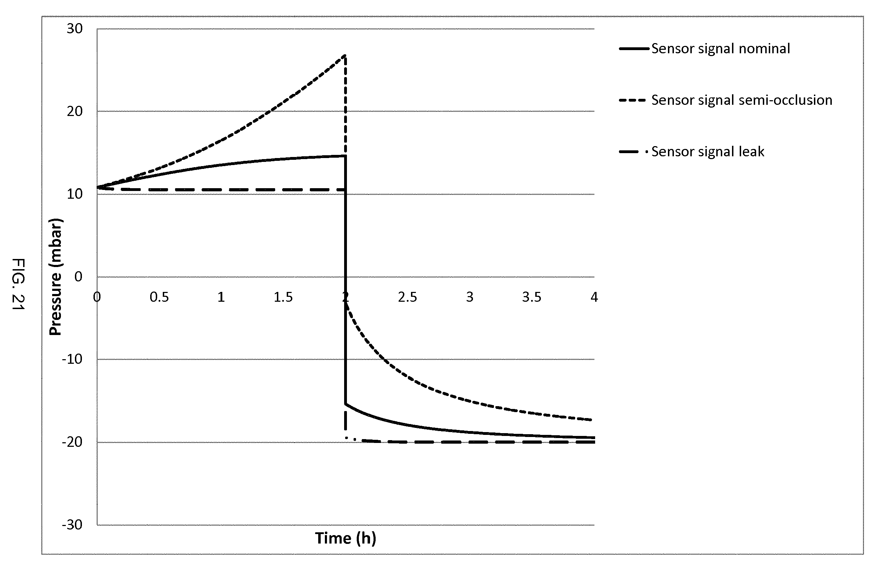

Figure 21: Evolution of the differential pressure sensor signal according to the test protocol given in Table 7, using simulation data given in Table 6.

## LIST OF ELEMENTS

| 1 | Membrane |
|---|---|
| 2 | Substrate |
| 3 | Inlet hole |
| 4 | Outlet hole |
| 5 | Flexible part |
| 6 | Mesa or rigid part |
| 7 | O-ring |
| 8 | Housing |
| 9 | Adjustment element |
| 10 | Cavity |
| 11 | Lower face |
| 12 | Upper face |
| 13 | Ball |
| 14 | Blade |
| 15 | Inlet of the medical device |
| 16 | Outlet of the medical device |
| 17 | Adjusting means |
| 18 | Outside the body |
| 19 | Inside the body |

## DETAILED DESCRIPTION OF THE INVENTION

In the following detailed description, reference is made to the accompanying drawings that form a part hereof, and in which are shown by way of illustration several embodiments of devices, systems and methods. It is to be understood that other embodiments are contemplated and may be made without departing from the scope or spirit of the present disclosure. The following detailed description, therefore, is not to be taken in a limiting sense. The invention is set forth and characterized in the independent claims, while the dependent claims describe other characteristics of the invention.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, any direction referred to herein, such as "top", "bottom", "left", "right", "upper", "lower", and other directions or orientations are described herein for clarity in reference to the figures and are not intended to be limiting of an actual device or system. Devices and systems described herein may be used in a number of directions and orientations.

As used herein, "have", "having", "include", "including", "comprise", "comprising" or the like are used in their open ended sense, and generally mean "including, but not limited to.

As used in this specification and the appended claims, the term "or" is generally employed in its sense including "and/or" unless the content clearly dictates otherwise.

The term "proximal" as used herein, is a broad term and is used in its ordinary sense, including, without limitation, near to a point of reference such as an origin or a point of attachment.

The term "distal" as used herein, is a broad term and is used in its ordinary sense, including, without limitation, spaced relatively far from a point of reference, such as an origin or a point of attachment.

The term "substantially" as used herein, is a broad term and is used in its ordinary sense, including, without limitation, being largely but not necessarily wholly that which is specified.

## Examples of medical device used by the method

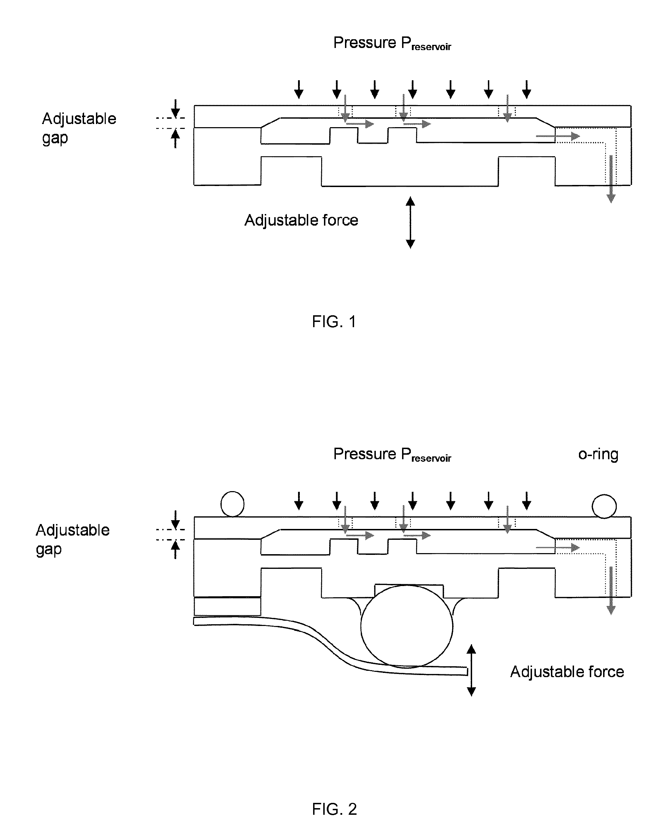

According to an embodiment but not limited to, the figures 1 and 2 show aspects of the valve used by a flow regulator. Said flow regulator may comprise a substrate (2) on top of which a membrane (1) is tightly fixed. Said regulator further comprises a cavity (10) which is arranged between the substrate (2) and the membrane (1). The distance between said substrate (2) and said membrane (1) corresponds to the depth of the cavity hereinafter called gap G. Said substrate (2) and/or said membrane (1) have through holes which are at least one inlet hole (3) and one outlet hole (4). Said holes permit the fluid to flow through said regulator. Inlet hole (3) and outlet hole (4) are in direct fluid communication with said cavity (10).

According to the same principle of the flow regulator, said membrane (1) comprises a lower face (11) which faces the cavity (10) and an upper face (12) on which the pressure of the fluid (also called P) is applied. Said membrane (1) is flexible in such a way that said membrane (1) comes into contact with said substrate (2) depending on the pressure of the fluid. Said contact may be total or partial, the main goal of this contact is to increase the fluidic resistance in such a way that the flow can be controlled even if the pressure of said fluid increases. When the pressure of the fluid increases, the depth of the cavity (gap G) decreases until the membrane (1) comes into contact (at least partially) to the substrate (2), thus the fluidic resistance increases for hindering said fluid flow.

The regulator may comprise several inlet holes (3). The holes positions and dimensions are arranged so that the fluid flow rate is passively regulated, depending on the fluid pressure, at least in a range of fluid pressure going from a first and at least a second predefined threshold values.

The figure 1 shows an embodiment with pillar. But the valve may have or not pillars. The pillar may be aligned to the inlet holes and may be located on the membrane and/or the rigid part. The position of the outlet may be located either on the top or the bottom or the side of the substrate or the membrane. Inlet and outlet may be on the same side. The position of the outlet could be arranged in the membrane or in the substrate.

Advantageously, the substrate (2) comprises a rigid part (6) surrounded by a flexible part (5). Due to the flexible part (5), said rigid part (6) is able to move downwardly and/or upwardly. In other words, the rigid part (6) can move to the membrane (1) or move away from the membrane (1) in such a way as to decrease or increase the gap G. Said rigid part may come into contact with said membrane.

In an embodiment, the regulator further comprises an adjustment means designed to move and/or to maintain at least temporarily said rigid part at a given position. Said adjustment means allows applying a force F on the rigid part of substrate to adjust the gap G of the cavity.

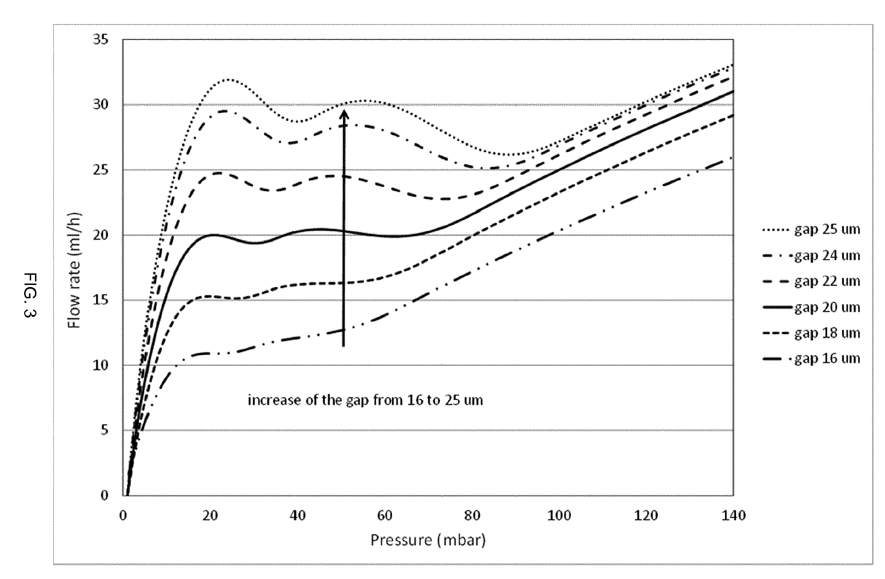

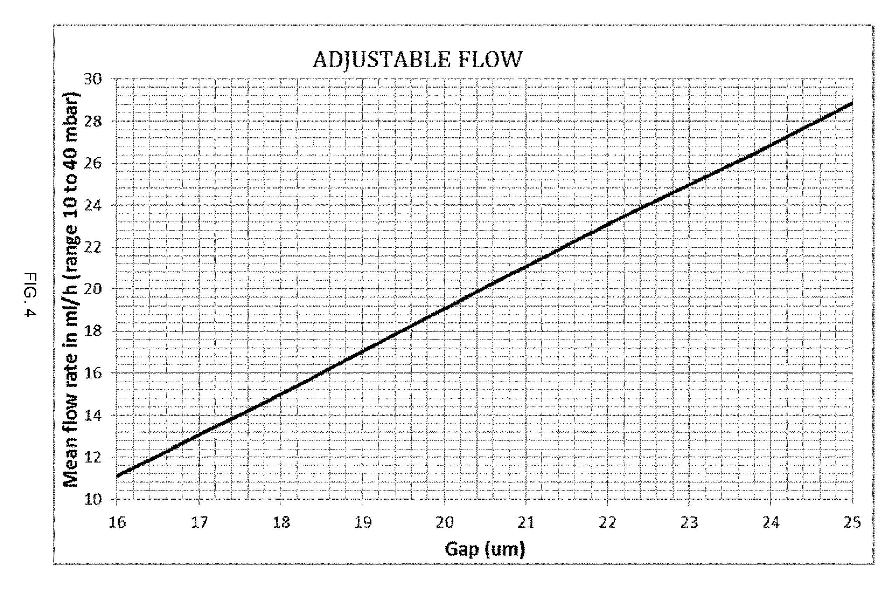

Thanks to said adjustment means, the gap G is adjustable, the graph of the figure 3 shows the effect of the gap on the flow rate. Thus, the changes of few microns around the nominal values of the gap induces an offset of the flow rate plateau while the pressure range of regulation is slightly shifted. The Figure 4 shows the means flow rate value, in the range of regulation 10 to 40 mbar, as a function of the gap. The linearity of this curve makes possible the use of a spring to adjust the flow rate. To achieve a displacement of only few microns, it is more relevant to control the force on the rigid part and not its displacement. Initially, the gap may be set at 25 um without deflection and pressure, the substrate being deflected only by compression force to simplify the assembly of the system.

In an embodiment, the membrane is much more flexible than the substrate. The pressure force transmitted by the membrane after contact with the substrate should be negligible compared to the restoring force of the substrate.

A force may be exerted by a spring, e.g. a flexible blade (14) in compression against a hard ball (13) glued on the mesa (6) as shown in Figure 2. The ball may prevent substrate flexure by transmitting no couple. The force may be adjusted by the rotation of an elliptic came (not shown in the figures) in contact with the blade or by adjusting the height of the blade support. A rotor (which may comprise two axially opposed magnets) may be used to change the setting (the gap between the substrate and the membrane and/or the force applying on the mesa). Said rotor may comprise several predetermined setting which may be externally changed by a specific tool (e.g. a magnetic lock).

Preferentially, strain gages in a full Wheatstone configuration are ideally implanted directly in the silicon membrane to form a differential pressure sensor. The sensor is connected to an electronic circuit comprising a coil to power externally the system, signal processing and wireless means to format and transfer the data to the end user (physician).

In one embodiment, a push-pull system may be made by gluing a flexible blade onto the mesa and by attaching the spring onto this blade. The blocking of the substrate due to a flexure may be prevented by the flexibility of the blade attached to the mesa. The spring and the flexible blade may be a single preformed blade.

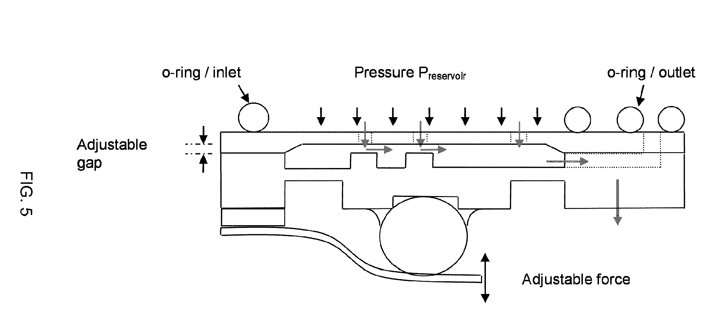

An adjustable valve (such shown by the figure 1, 2, 5) may be arranged in a medical device comprising a housing (8), an inlet (15) adapted to be inserted in the cranial cavity and an outlet (16) adapted to be inserted in the other cavity. The medical device may comprise (upstream the adjustable valve) advantageously a check valve (e.g. a ball valve) and/or a reservoir to flush the medical device. The medical device may comprise a safety valve that opens at high pressure (e.g. ball valve).

Example of numerical simulations

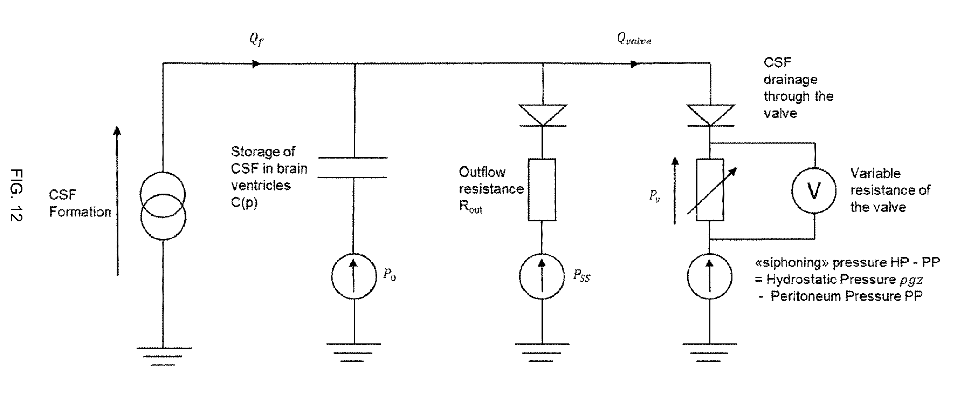

An equivalent electrical model, based on the original study of Marmarou, has been used to simulate cerebrospinal fluid dynamics. A variable resistance has been added to simulate the shunt itself. The figure 12 shows an example of an equivalent electrical model for CSF dynamics analysis of a patient with a shunt.

The CSF continuously produced by the patient is either stored in the ventricles or reabsorbed via the arachnoid villi or drained through the valve toward the peritoneum cavity. We do not consider here the fluctuations of ICP induced by the vasogenic system. For the sake of clarity, we assume first that the residual outflow resistance is infinite, the CSF being only diverted towards the peritoneum in steady state. The evolution of the pressure in the brain is derived by solving a differential equation, based on the diagram provided in Figure 1, with the Euler's method.

The numerical constants used in the simulations are provided in Table 1:

Table 1: numerical constants for the simulation of the active tuning of a hydrocephalus valve.

| Pss (mbar) | 10 |
|---|---|
| Po (mbar) | 6.6 |
| PP (mbar) | 0 |
| Rout (SI) | Inf. |
| z max (cm) | 30 |
| E (1/mL) | 0.184 |
| Pbo (mbar) | 10.8 |
| dt (s) | 1 |

We consider a flow regulator set at 20 ml/h, the initial value of the ICP being equal to 10 mbar. In case of a sudden increase of the production rate, from 20 to 22 ml/h at t=0, the evolution of the ICP with time could be simulated as reported in the figure 13. The scenario for the different postural change of the patient is summarized hereafter (Table 2):

Table 2: description of the different postural changes of the patient with time.

| Time constant | value (h) | Postural change |
|---|---|---|
| t0 | 0 | decubitus to upright |
| t1 | + 16h | upright to decubitus |
| t2 | + 24h | decubitus to upright |
| t3 | + 40h | upright to decubitus |

(continued)

| Time constant | value (h) | Postural change |
|---|---|---|
| t4 | + 48h | decubitus to upright |

The time t0 and t2 could correspond for instance to two successive patient's waking at 7 am while the time t1 and t3 could correspond to two successive patient's bedtime at 11 pm.
Unacceptable increase of ICP due to underdrainage is observed, in particular during the night (decubitus). This curve illustrates the interest in fine tuning the valve setting after implant.

A reference curve corresponding to the typical evolution of the pressure (for example ICP) in case of postural change, following the scenario of Table 2, but without increase of the nominal flow rate which remains equal to 20 mL/h, is provided in the figure 13 bis. As expected, the ICP is unchanged.

The evolution of ICP, for a decrease of the production rate of 2 ml/h at t=0, leading to Qf = 18 ml/h, are provided in the figure 14, using the same scenario than previously for the different postural changes. In that case overdrainage is observed during the main part of the day. Even if the large fluidic resistance of the flow regulator strongly limit the effect of this overdrainage on the ICP value, long term complications to such low mean ICP value could be expected.

The following simulation is based on the numerical simulation described above.

## Method of setting of the regulator

We work on the principle that the pressure gradient monitored by the pressure sensor is equal to the difference of pressure between the top surface of the membrane (assumed to be equal to ICP) and the bottom surface of the membrane which is in pressure communication with the peritoneum. Thus, we assume the following rule:

$$\Delta P = ICP + HP - PP$$

Where ICP is the intra-cranial pressure, HP the hydrostatic pressure and PP the peritoneum pressure. Ideally, to set a hydrocephalus valve, the physician is mainly interested in the knowledge of the ICP and the flow rate (of CSF production rate) but the ICP cannot be measured by the sensor of our device (because our sensor is not an absolute pressure sensor). Other patient dependent parameters as the residual drainage resistance Rout may be used and will be discussed hereafter.

As a general trend, the present invention is based on the combination of device setting change with patient postural change to make fast and reliable valve adjustment using the integrated pressure sensor. Furthermore, this new method is based on the use of a pressure gradient sensor, not to adjust the pressure at which the anti-siphon valve closes but to adjust the desired flow rate of the device. Thus, this new method consists in the combination of the change of the device setting (e.g. flow rate) with patient postural change. The monitoring and the analysis of the pressure gradient values during these changes allows the tuning of the device in accordance with the patient physiological characteristics.

For example, for a device initially set at 20 ml/h, and for a patient generating 25 ml/h of CSF, the differential pressure sensor will indicate a very large value (typically > 20 mbar). For a patient generating 15 ml/h, the differential pressure measured will be small (typically < 10 mbar).

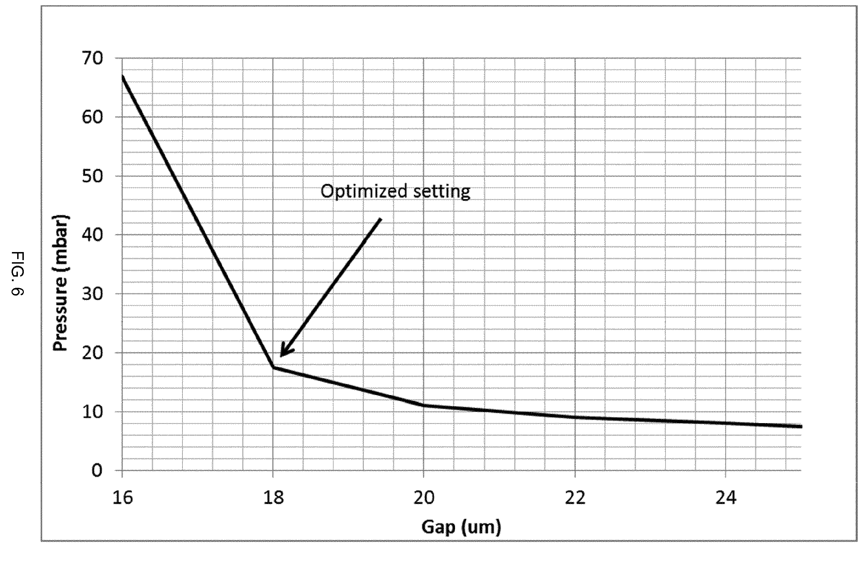

According to one embodiment, the figure 6 shows the differential pressure monitored by the sensor as a function of the device setting (here referred as a gap in $\mu$m, each setting position corresponding to a given gap). Here, the gap is the distance between a surface of the mesa and a surface of the membrane. For this example, the CSF production rate is 15 ml/h. According to one embodiment, a change of setting can adjust the gap by step equivalent to change of about 5% of the nominal flow rate. We note "I" a setting position, I0 is the position that leads to a determined mean flow rate for example 20 ml/h. Thus, $I_{0+1}$ is the setting that leads to 21 ml/h while the setting $I_{0-1}$ corresponds to 19 ml/h, and so on.

Optionally, the initial setting of the device correspond to a high flow rate setting (for example the greatest gap corresponding to the maximum flow rate of the device) so as to drain the excess of CSF in the patient's brain. Preferentially after the surgery, the patient stays in decubitus during several hours and the ICP stabilizes at around 10 mbar (for example).

A first method may comprise the following steps:

• Place the patient in upright position,
• Monitor the evolution of the pressure with time dP/dt
• Reduce the device setting I until the sign of dP/dt becomes positive.

We note $I_k$ the setting which allows changing the sign of time derivative dP/dt. Thus, the optimal setting is comprised between $I_k$ and $I_{k+1}$.

A second method may comprise the following steps:

- Set the device at $I_{0+j}$ where $I_{0+j}$ may be the initial setting (for example after implant),
- Set the device at $I_{0-j}$,
- Place the patient in upright position,
- Monitor the evolution of the pressure with time dP/dt
- Increase the device setting until the sign of dP/dt becomes negative.

With this method, the optimal setting is comprised between $I_k$ and $I_{k-1}$.

A third method may consists in changing setting alternatively in positive and negative direction with respect the foreseen nominal value $I_0$, in order to limit the change of ICP during the adjustment procedure. Indeed the ICP alternatively increases and decreases, limiting the overall change of the mean ICP value. The third method may comprise the following steps (j is the range of setting that will be used to adjust the device, k is the step of the setting search and n is the iterative number of adjustment steps):

- Set the device at $I_{0+j}$ where $I_{0+j}$ may be the initial setting with n=0 (for example after implant),
- Place the patient in upright position,
- Monitor the evolution of the pressure with time dP/dt.
- If dP/dt stays positive, use a j value larger in order to get the right sign of dP/dt,
- Set the device at $I_{Nominal} + (j - k \times n) \times (-1)^n$ using n=1; if dP/dt becomes negative set the device at

$$I_{Nominal} + (j - k \times n) \times (-1)^n$$

using n=2 and so on.

The optimal setting is obtained when the change of setting of one k unit change the sign of dP/dt.

To check out that the setting is correct, the pressure after a long stay in upright position shall be in a determined range for example 13 +/- 6 mbar.

The method is still valid if a setting change does not correspond exactly to a fixed value of flow rate change. The mandatory requirement is just that an increase/decrease of the actual setting I will induce an increase/decrease of the mean flow rate.

As a general trend, the analysis of the sign of dP/dt is valid essentially when the pressure gradient is comprised in its regulation range, or in other words the functioning point of the device is located onto the "plateau", where the flow is expected to be constant. The different methods are therefore more effective when the patient is in vertical or tilted position. The analysis of the evolution of pressure in case of partial or total occlusion is discussed hereafter.

**Simulation of setting using the method 1**

We consider a valve having a nominal flow rate $Q_0$ of 20 mL/h for the setting 0, the increase/decrease of 1 setting corresponding to an increase/decrease of 1 mL/h for the nominal flow rate through the valve (e.g. $Q_2$ = 22 mL/h and $Q_{-4}$ = 16 mL/h). We assume now that the patient exhibits a production rate of 22 mL/h, while the other constants necessary to perform the simulations are given in Table 1.

After implant the patient is in decubitus with the valve wide open in order to reduce the ICP to a value slightly lower than its basal pressure $P_b$. The patient is then placed in upright position while the setting is decreased until stabilization and finally an increase of the pressure, the best setting being here the penultimate one.

After the stabilization period at the best setting the patient comes back in decubitus to check out that the ICP is not affected by postural change and that its value is within the initial range estimated during an Infusion Test.

A typical protocol is given in the Table 3.

Table 3: Adjustment protocol for the determination of the best setting of the valve using the method 1.

| Time constant | value | Valve setting | Patient's position |
|---|---|---|---|
| t0 | 0h | +5 | decubitus |
| t1 | + 12h | +4 | upright |
| t2 | + 12h45 | +3 | upright |

(continued)

| Time constant | value | Valve setting | Patient's position |
|---|---|---|---|
| t3 | + 13h30 | +2 | upright |
| t4 | +14h15 | +1 | upright |
| t5 | + 15h | +2 | decubitus |

The ideal setting of the device is the setting +2, leading to a flow rate through the valve equal to the production rate. The evolutions of the ICP and the sensor signal during the valve tuning are provided in the figure 15. The change of the slope is an indication that the new setting is too low. The final postural change of the patient is used to check the effectivity of the anti-siphoning effect of the device in case of correct valve tuning.

**Simulation of setting using the method 2**

We still assume a production rate of 22 mL/h. This production rate is estimated during an Infusion Test. Therefore we are able to limit the range of setting during the tuning at +/-3 around this estimated value. The tuning procedure is summarized in the table 4. The best tuning is +2.

To increase the change of pressure during the change of setting, the patient is ideally placed in upright (resp. decubitus) position during an increase (resp. decrease) of the setting.

Table 4: Adjustment protocol for the determination of the best setting of the valve using the method 2 (best Setting = +2).

| Time constant | value | Valve setting | Patient's position |
|---|---|---|---|
| t0 | 0h | +5 | decubitus |
| t1 | + 12h | -1 | decubitus |
| t2 | + 12h45 | +1 | upright |
| t3 | + 13h30 | +3 | upright |
| t4 | + 14h15 | +2 | upright |
| t5 | + 15h | +2 | decubitus |

**Simulation of setting using the method 3**

We still assume a production rate of 22 mL/h. This production rate is estimated during an Infusion Test. Therefore we are able to limit the range of setting during the tuning at +/-3 around this estimated value. The tuning procedure is summarized in the table 5. The best tuning is +2. To increase the change of pressure during the change of setting, the patient is ideally placed in upright (resp. decubitus) position during an increase (resp. decrease) of the setting.

Table 5: Adjustment protocol for the determination of the best setting of the valve using the method 2 (best Setting = +2).

| Time constant | value | Valve setting | Patient's position |
|---|---|---|---|
| t0 | 0h | +5 | decubitus |
| t1 | + 12h | -1 | decubitus |
| t2 | + 12h45 | +3 | upright |
| t3 | + 13h30 | +1 | decubitus |
| t4 | + 14h15 | +2 | upright |
| t5 | + 15h | +2 | decubitus |

**Modelling the arterial pressure oscillations**

In order to get a more realistic picture of the pressure trend during the device setting, the change of the cerebral artery volume due to heart action shall be considered. It is assumed that this volume change is balanced by the same CSF volume fluctuations.

The production rate of CSF includes therefore an additional term in cos($\omega t$):

$$Q_f = Q_{f0} + \omega C_A \delta P_A \cos(\omega t)$$

Where $\omega$ is the heart rate angular frequency, $C_A$ the cerebral artery compliance and $\delta P_A$ the amplitude of the sinusoidal perturbation:

$$P_A(t) = P_{A0} + \delta P_A \sin(\omega t)$$

The numerical constants used in the simulations are provided in the table 6 while the adjustment protocol the given in the table 5:

Table 6: simulations constants for modelling the effect of cerebral artery compliance on ICP.

| | |
|---|---|
| Pss (mbar) | 10 |
| Po (mbar) | 9 |
| PP (mbar) | 0 |
| $R_{out}$ (mmHg/mL/min) | 13 |
| z max (cm) | 30 |
| E (1/mL) | 0.2 |
| Pbo (mbar) | 14 |
| $\omega$ ($s^{-1}$) | 6.28 |
| $E \times C_A \times \delta P_A$ | 0.05 |
| dt (s) | 0.05 |

The evolution of the ICP after the 12h of ICP stabilization is provided in the figure 18. The adjustment methods are not affected by the sinusoidal trend of the ICP. Low-pass filter may be used for ICP profile analysis.

**Method of detecting a failure**

**In case of total occlusion:**

Two methods may be used: the DC analysis method (stationary) and/or the AC analysis method.

**DC analysis method**

If the total occlusion is located in the proximal (i.e. before the valve) and/or distal (i.e. after the valve) catheter, the pressure between the top and bottom surfaces of the membrane will equilibrate, leading to a pressure gradient equal to zero in both positions (horizontal or vertical position). After stabilization, we have: $\Delta P_h - \Delta P_v = 0$

For an occlusion near the device (i.e. proximal or just after the device outlet), we do not expect a change of pressure by postural changes. But, an occlusion at the end of the distal catheter induces a sudden increase of pressure and then quickly relax to zero. In that latter case, a total liquid volume corresponding to the change of volume of the fluidic line upward the regulator will pass through the device, inducing a movement of the membrane which can be detected by the sensor.

Thanks to the elasticity of the fluidic line it could be possible to discriminate between proximal or distal occlusion if this latter occlusion takes place just after the device (i.e. the valve) and not at the end of the distal catheter. But, if the elasticity of the proximal cavity (in communication with the top surface of the flexible membrane of the valve) is small, the pressure will remain equal to zero during the postural change of the patient and the possibility to distinguish between proximal and distal occlusions is lost.

Thus, a total occlusion diagnosis can be detected or confirmed by these observations. The method for detecting an occlusion may comprise:

- Provide a medical device adapted to regulate a fluid which flows from the cranial cavity to another cavity of the body patient, the medical device comprising:

  ◦ An inlet located in the cranial cavity, in which the fluid enters in the medical device
  ◦ An outlet located in the other cavity, by which the fluid flows out the medical device
  ◦ A pressure sensor which senses the differential pressure between the cranial cavity and the other cavity

- Record a first differential pressure measurement when the patient is in decubitus or upright position,
- Change the position of the patient
- Record a second differential pressure measurement,
- Compare the first and the second differential pressure data
- Detect an occlusion based on this comparison.

A purge may be necessary to release this occlusion. The purge is made possible by a filling port that is externally accessible via a syringe.

**AC analysis method**

The total occlusion may be detected by analyzing the AC signal of the ICP pressure (for example via a mathematical model). The AC component of ICP due to change of blood volume in the brain are no longer transmitted to the device for proximal occlusion. This method is the best way to distinguish proximal and distal occlusion.

The method for detecting an occlusion may comprise:

- Provide a medical device adapted to regulate a fluid which flows from the cranial cavity to another cavity of the body patient, the medical device comprising:

  ◦ An inlet located in the cranial cavity, in which the fluid enters in the medical device
  ◦ An outlet located in the other cavity, by which the fluid flows out the medical device
  ◦ A pressure sensor which senses the differential pressure between the cranial cavity and the other cavity

- Record a first set of measurements,
- Process the first set of measurements,
- Detect an occlusion based on the first set of measurements.

**In case of partial occlusions:**

**If the partial occlusion is located inside the valve:**

Because the device shows the smallest restriction in the fluidic pathway between the brain and the peritoneum, this kind of occlusion have the highest probability to occur. These restrictions are the holes in the membrane and the gap between the membrane and the pillars. Any occlusion in the device (e.g. located in the holes) leads to an increase of the measured value of the pressure gradient through the device.

For a given device correctly adjusted to the patient CSF production rate, this kind of occlusion will lead to an increase of the pressure at least equivalent to a change of position because of the translation of the Q(P) curve towards the low flow rate. This increase of pressure can be observed in decubitus, and a change of position will increase again the measured value.

The method for detecting an occlusion may comprise:

- Provide a medical device adapted to regulate a fluid which flows from the cranial cavity to another cavity of the body patient, the medical device comprising:

  ◦ An inlet located in the cranial cavity, in which the fluid enters in the medical device

◦ An outlet located in the other cavity, by which the fluid flows out the medical device
◦ A pressure sensor which senses the differential pressure between the cranial cavity and the other cavity

- Record a first differential pressure measurement when the patient is in decubitus or upright position,
- Change the position of the patient
- Record a second differential pressure measurement,
- Compare the first and the second differential pressure data
- Detect an occlusion based on this comparison.

A purge may be necessary to flush the device from any protein residual.
A new valve adjustment may be required after detection of partial occlusion.

**If the partial occlusion is located in proximal and/or distal catheter:**

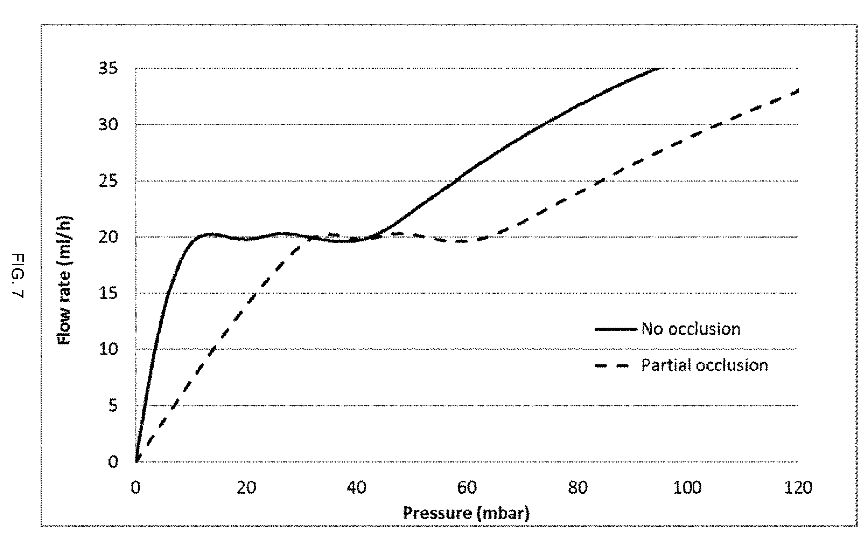

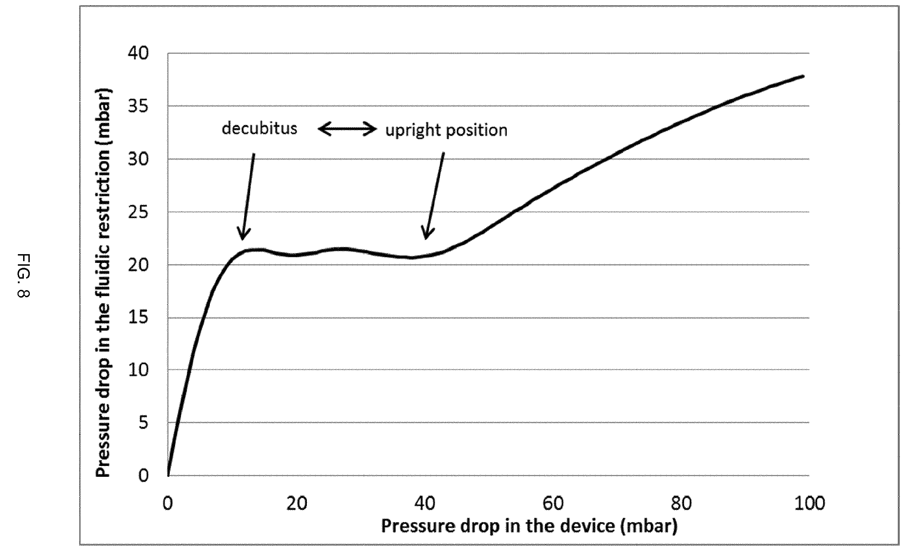

We consider now a partial occlusion equivalent to a fluidic resistance of 3.8E11 Pa.s/m3 (for example). The change of the device characteristics is shown Figure 7. The nominal regulated flow rate is equal to 20 ml/h in both cases, there is just a shift induced by the additional fluidic resistance. We consider a patient having a normal ICP of 10 mbar. In case of partial occlusion equivalent to said fluidic resistance of 3.8E11 Pa.s/m3, the ICP rises up to 30 mbar but the sensor only measures 10 mbar which corresponds to the pressure drop through the device. By design, the device is "blind" to partial occlusion if the device is originally well adjusted to the effective CSF production rate of the patient. The pressure drop in this fluidic restriction versus the pressure drop in the device itself is shown Figure 8. When the patient moves from decubitus to upright position, the anti-gravity properties of the device are still active with a partial occlusion and the sensor signal is not affected by partial occlusion: the pressure drop in this restriction is constant during the movement while the pressure drop through the device has increased of 30 mbar which corresponds to the hydrostatic pressure HP.
In normal situation the sensor shall experience a negative pressure while in case of partial occlusion, considering the former case with an ICP of 30 mbar induced by said occlusion, the pressure will equilibrate and the sensor will not measure this negative pressure.
A first approach consists in using a tilting chair and placing the patient not at -90° but at an angle comprised between -10° to -60° and to analyse the decrease of the measured pressure as a function of this angle, after a stabilization period.
Other methods to confirm the hypothesis of a partial occlusion may be used, for example a change of setting or a AC method.

*"Change of setting" methods*

The typical patient characteristics (E, po and Rout) have been determined before or after the shunt implantation to characterize the CSF dynamics of the patient. Where E is the cerebral elasticity or elastance coefficient, po is the pressure in the extradural venous system and Rout is the residual drainage resistance. The initial p(t) curve could be therefore used as a reference.
In order to check the eventual presence of an occlusion it is possible to use the method described previously consisting in changing the setting of the device and monitoring the evolution of the pressure.
In case of occlusion the pressure measured by the sensor is no longer equal to the ICP even when the patient is in decubitus and when we neglect the peritoneum pressure.
In case of partial occlusion that leads to an increase of ICP, we expect a change of the CSF dynamics because of the decrease of the brain compliance at high ICP.
The fitting of the curve p(t) during the test of setting change will give the best coefficient of determination $R^2$ for a reduced value of E.
Moreover, in case of occlusion, the slope of the curve Q(P) before and after the plateau will decrease. This decrease will result in a change of the typical curve p(t) during a test according to the previous method.
The decrease of this slope induces an increase of the measured variation of pressure during these tests by a factor proportional to the ratio of the ICP.
It is therefore possible to extract the real value of the ICP by doing a simple rule of three: we use a +1 method consisting in changing the position setting of the device of +1 (to increase of 1 unit the nominal setting of the device).
The pressure change after stabilization is proportional to $p_b(i)$:

$$p(t = \infty) = p_b(i+1) = \left(\frac{Q_{\mathrm{valve}}(p_b(i), i)}{Q_{\mathrm{valve}}(p_b(i), i) + \delta Q}\right) p_b(i) = \frac{p_b(i)}{1 + \dfrac{\delta Q}{Q_{\mathrm{valve}}(p_b(i), i)}}$$

If the partial occlusion leads to an increase of the ICP of a factor 3, the relative change of pressure during the change of setting +1 will be also increased by a factor 3 according to the reference value recorded just after implantation.

In brief: a distal or proximal occlusion will be therefore detected by analysing the curve p(t) and by comparing this curve with a reference curve obtained just after implantation.

The method for detecting an occlusion may comprise:

- Provide a medical device adapted to regulate a fluid variable which flows from the cranial cavity to another cavity of the body patient, the medical device comprising:

    ◦ An inlet located in the cranial cavity, in which the fluid enters in the medical device
    ◦ An outlet located in the other cavity, by which the fluid flows out the medical device
    ◦ An adjustable valve in fluid communication with the inlet and the outlet of the medical device, said adjustable valve being adapted to be set by a caregiver in such a way as to adjust the fluid variable,
    ◦ A pressure sensor which senses the differential pressure between the cranial cavity and the other cavity

- Change the fluid variable according to a new setting,
- Monitor over time the differential pressure,
- Analyze the curve of the monitored differential pressure (for example: compare the curve of the monitored differential pressure to reference data)
- Determine an occlusion based on this comparison.

*AC method*

According to Lemaire J.J. et al., Slow Pressure Waves in the Cranial Enclosure, Acta Neurochir 144: 243-254 (2002), the analysis of the slow pressure waves in the cranial cavity could be also used to for assessment of CSF disorders.

Just after implantation, typical AC components of the ICP signal will be recorded as reference for standard ICP, when the device is correctly adjusted to the patient production rate of ICP.

A decrease of the measured AC amplitude of the pressure could be attributed to an increase of the ICP. This change of the AC amplitude could be correlated to the former test to confirm the presence of a partial occlusion.

The method for detecting an occlusion may comprise:

- Provide a medical device adapted to regulate a fluid which flows from the cranial cavity to another cavity of the body patient, the medical device comprising:

    ◦ An inlet located in the cranial cavity, in which the fluid enters in the medical device
    ◦ An outlet located in the other cavity, by which the fluid flows out the medical device
    ◦ A pressure sensor which senses the differential pressure between the cranial cavity and the other cavity

- Record a first set of measurements,
- Process the first set of measurements,
- Detect an occlusion based on the first set of measurements.

**In case of leakage**

According to the device design, we should consider two kinds of leakage:

• a leakage due to a membrane crack or
• a leakage due to particle between the pillars and the membrane leading to a permanent gap (valve leakage).

**In case of a leakage due to a membrane crack :**

By design the silicon membrane of the device is expected to sustain pressure of several bars. In case of a shock on the patient head, the pressure sensor could be used to detect a mechanical failure of the membrane.

The pressure sensor is preferably made of a strain gauge implanted directly in the silicon membrane. The detector circuit at the surface of the membrane could be therefore considered as a crack-guard: any crack in the membrane will lead to an open detector circuit.

**In case of valve leakage:**

By design the device is not sensitive to small particles (lower than 2 um in diameter). In case of contamination with larger particles or residuals (proteins...), we expect an increase of the derivation flow through the valve for a given ICP.

In a first approximation we assume that this permanent gap is equivalent to a change of the device setting that leads to an increase of the nominal flow rate.

If typical symptoms of overdrainage are observed, the first analysis method consists in doing an adjustment of the device with a monitoting of the p(t) curve during this process.

An abnormal increase of the nominal flow rate should be rather attributed to a valve leakage rather than an increase of CSF production if this phenomenon is sudden and permanent.

The method for detecting an valve leakage may comprise:

- Provide a medical device adapted to regulate a fluid variable which flows from the cranial cavity to another cavity of the body patient, the medical device comprising:

    ◦ An inlet located in the cranial cavity, in which the fluid enters in the medical device
    ◦ An outlet located in the other cavity, by which the fluid flows out the medical device
    ◦ An adjustable valve in fluid communication with the inlet and the outlet of the medical device, said adjustable valve being adapted to be set by a caregiver in such a way as to adjust the fluid variable,
    ◦ A pressure sensor which senses the differential pressure between the cranial cavity and the other cavity

- Change the fluid variable according to a new setting,
- Monitor over time the differential pressure,
- Analyze the curve of the monitored differential pressure (for example: compare the curve of the monitored differential pressure to reference data)
- Determine an valve leakage based on this comparison.

**Simulation of failure analysis**

We assume first that the volume displaced by the flexible membrane per unit of pressure is large and almost constant over the range of pressure 10 to 40 mbar. In that case, we show in the figure 19 the evolution of the pressure sensor signal in various cases, comprising notably either proximal or distal occlusion. We assume occlusion in the proximal catheter or at the entry of the distal catheter (i.e. just after the valve itself). The water column due to the distal catheter in vertical position has an effect on the sensor signal depending on the localization of the occlusion.

For a proximal occlusion, we expect a first peak of pressure when the patient moves from decubitus to upright position. This effect is due to the compliance of the proximal cavity that will be contracted by the negative pressure generated by the water column. A linear relaxation is then observed as shown in the figure 19. This linear behavior is related to the fact that the flow rate induced by the movement of the flexible membrane is constant.

We consider the presence of a distal occlusion in a device that exhibits a small compliance: the flexible membrane will remain all the time at equilibrium and there is no possibility to localize the occlusion using this method. The analysis of the short-term oscillations of the sensor signal is then required: in case of proximal occlusion a strong reduction of pressure oscillations due to the vasogenic system is expected.

Typical example considering a residual drainage function via arachnoid vili and variation around this value to simulate partial occlusion and leakage: the simulation data are summarized in the table 7. The initial ICP value is assumed to be equal to Pb0 (i.e. 10.8 mbar).

Table 7: Simulation data for failure analysis purpose.

| | |
|---|---|
| $P_{ss}$ (mbar) | 10 |
| $P_o$ (mbar) | 6.6 |
| PP (mbar) | 0 |
| $R_{out}$ nominal (SI) | $3.6^E11$ |

(continued)

| | |
|---|---|
| $R_{out}$ partial occlusion (SI) | 3.6$^E$12 |
| $R_{out}$ leakage (SI) | 3.6$^E$10 |
| z max (cm) | 30 |
| E (1/mL) | 0.184 |
| Pbo (mbar) | 10.8 |
| dt (s) | 1 |

In the table 8 is provided a typical test protocol for failure analysis.

Table 8: Test protocol for failure analysis.

| Time constant | value | Valve setting | Patient's position |
|---|---|---|---|
| t0 | 0h | -5 | decubitus |
| t2 | + 2h00 | 0 | upright |

The evolution of the intracranial pressure is provided in the figure 20 while the evolution of the pressure monitored by the differential sensor is given in the figure 21.
For sake of clarity, the values of Rout corresponding to Nominal, Leakage and Semi-occlusion curves are here significantly different (one order of magnitude).
The analysis of the pressure trends is straightforward. In case of leakage the partial closing of the valve (setting -5) is not able to generate underdrainage while in case of semi-occlusion a large increase of the pressure is observed.

**Method for a non-invasive characterization of the CSF dynamics**

In a general manner, any information about CSF flow or pressure is fundamental to diagnose and characterize cerebral disease. In case of abnormal function of the cerebrospinal fluid outflow system, several methods are described in the prior art part of this document.

**Standard model for CSF dynamics**

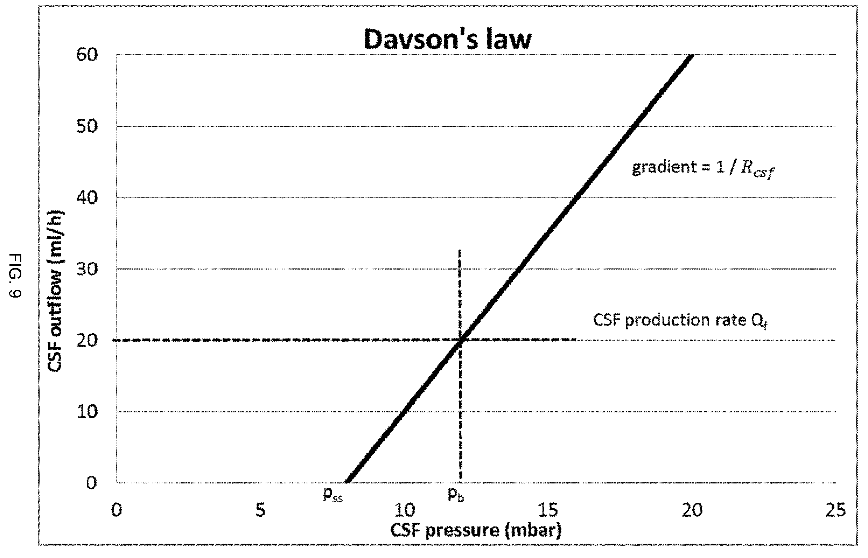

We do not consider fluctuation due to periodic changes of the cerebral arterial blood volume and changes of venous outflow generated by respiration.
We assume a constant production rate of CSF. Therefore the production of CSF shall be balanced between storage and absorption. This absorption $Q_{abs}$ is proportional to the difference of pressure between the intracranial pressure p=ICP and the pressure in the sagittal sinuses $p_{ss}$ (from Davson's law, see the figure 9):

$$Q_{abs} = \frac{p - p_{ss}}{R_{out}}$$

Where Rout is the resistance to CSF absorption.

The storage of CSF is proportional to the cerebrospinal compliance C(p) and the rate of change of CSF pressure $\frac{dp}{dt}$:

$$Storage = C(p)\,\frac{dp}{dt}$$

The compliance of the cerebrospinal space C(p) is constant up to given pressure $p_{opt}$ and increases when the CSF pressure is larger than $p_{opt}$:

$$C(p) = \frac{1}{E(p - p_0)} \quad for \ p > p_{opt}$$

Where E is the cerebral elasticity or elastance coefficient which is patient specific. The compliance of the brain decreases for high ICP. By writing the conservation of matter we finally derive:

$$\frac{1}{E(p(t) - p_0)} \frac{dp(t)}{dt} + \frac{p(t) - p_b}{R_{out}} = Q_{ext}(t)$$

Where $Q_{ext}(t)$ is the rate of external volume addition; the term $p_b$, which is the baseline CSF pressure (resting pressure), is used in the latter formula instead of $p_{ss}$ which is difficult to measure.

This equation could be solved easily in case of constant infusion rate test with $Q_{ext}(t) = Q_{const}$:

$$p(t) = p_0 + \frac{\left(\frac{p_b - p_0}{R_{out}} + Q_{const}\right)(p_b - p_0)}{\frac{p_b - p_0}{R_{out}} + Q_{const}\left(e^{-E\left[\frac{p_b - p_0}{R_{out}} + Q_{const}\right]t}\right)}$$

For a bolus injection of $\Delta V$, the evolution of the pressure becomes:

$$p_b = p_0 + \frac{(p_b - p_0)e^{E\left[\Delta V + \left(\frac{p_b - p_0}{R_{out}}\right)t\right]}}{1 + e^{E\Delta V}\left(e^{E\left[\frac{p_b - p_0}{R_{out}}\right]t} - 1\right)}$$

This bolus injection is usually used to determine the pressure volume index PVI:

$$PVI = \frac{\Delta V}{log\left(\frac{p_p - p_0}{p_b - p_0}\right)}$$

Where $p_p$ is the peak pressure just after the bolus injection [5].

According to Marmarou et al. (Compartmental analysis of compliance and outflow resistance of the cerebrospinal fluid system, J. Neurosurg. 43, 523534 (1975)) and Avezaat C.J.J. et al. (Clinical observations on the relationship between cerebrospinal fluid pulse pressure and intracranial pressure, Acta Neurochirurgica 79, 13-29 (1986)), the evolution of the intracranial pressure for increasing volume has an exponential form:

$$p = (p_b - p_0)e^{E\Delta V} + p_0$$

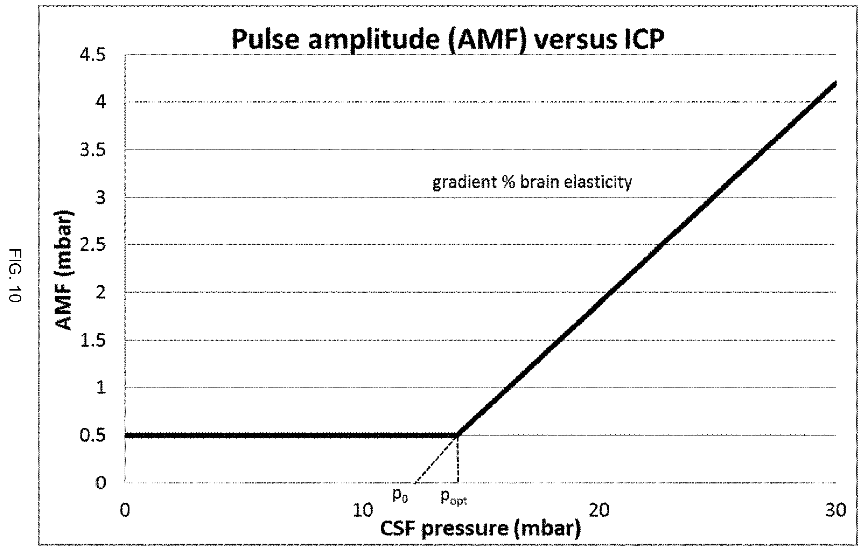

Because the craniospinal space is enclosed in the rigid skull, the compliance is small, typically from 0.1 to 0.3 ml$^{-1}$ (according to Cieslicki, K., Mathematical modelling of the infusion test, Pol. J. Med. Phys. Eng 13: 33-54 (2007)). The former relationship is useful to determine the pulse amplitude (AMF) of CSF due to the change of the blood volume in the brain after heart contraction, since this abrupt change of volume is similar to a bolus injection (see the figure 10):

$$AMP + p_p - p_b = (p_b - p_0)(e^{E\Delta V} - 1)$$

The analysis of the slow pressure waves in the cranial cavity could be also used to for assessment of CSF disorders (according to Lemaire J.J. et al., Slow Pressure Waves in the Cranial Enclosure, Acta Neurochir 144: 243-254 (2002)).

Eklund A. et al. (Assessment of cerebral fluid outflow resistance, Med. Bio. Eng. Comput. 45, 719-735 (2007)) reviewed the different methods of investigation of the CSF dynamics by active infusion of artificial CSF and pressure recording. After implantation of a shunt, these methods are still used to verify the good functioning of the device (according to Weerakkody et al., Clinical assessment of cerebrospinal fluid dynamics in hydrocephalus; Guide to interpretation based on observational study, Acta Neurol. Scand. 124, 85-98 (2011)).

Because these methods usually require the placement of two needles in the lumbar canal, an in vivo (but non-invasive, i.e. without needle) determination of the shunt functionality is desirable. Methods for the in vivo adjustment of the shunt to the patient production rate of CSF are also required.

**New method for a non-invasive characterization of the CSF dynamics**

The new method is related to the analysis of the pressure sensor signal to determine physiological parameters of the CSF dynamics of the patient. By adding an auto-regulated shunt in the CSF fluidic pathway, we should introduce a new term in the equation of the conservation of the matter:

$$Q_f + Q_{ext} = Q_{abs} + Q_{storage} + Q_{valve}$$

Where $Q_f$ is the formation rate of CSF, $Q_{ext}$ is the externally injected flow of liquid, $Q_{abs}$ is the absorbed rate of CSF through the arachnoid villi, $Q_{storage}$ is the rate of liquid storage due to pressure change and $Q_{valve}$ the flow of CSF through the valve.

We note:

p = intracranial CSF pressure
$p_{ss}$ = sagittal sinuses pressure
po = pressure in the extradural venous system
$p_b(i)$ = baseline ICP pressure, at rest in decubitus, for the setting i
$Q_{valve}(p_b(i), i)$ = flow through the valve at equilibrium, for the setting i
Rout = fluidic resistance to CSF outflow towards physiological routes
$\Delta P_{reg}$ = pressure range wherein the regulated flow is constant
PP = peritoneum pressure

We assume first that the compliance of the shunt is very small compared to the brain itself. To simplify the formalism of the evolution of the pressure with time, we assume that the peritoneum pressure PP is equal to zero. The real gradient of pressure through the valve is reduced of PP (typically 2 mbar). An example of a full model including PP is given in annex.

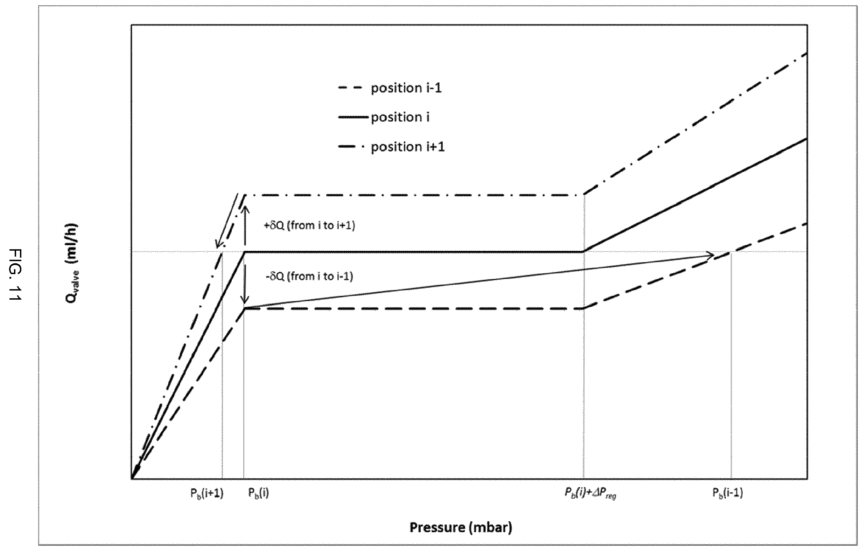

A simplified view of the CSF flow through the regulator is shown in the figure 11. The device after adjustement is assumed to be in position i with a flow rate at rest of $Q_{valve}(p_b(i), i)$. The valve fluidic characteristics after change of the device selector position of +/- 1 step are also shown. We note $\delta Q$ the absolute change of the nominal flow rate of the device in these two new positions.

At rest, the former equation reduces to:

$$Q_f = \frac{p_b(i) - p_{ss}}{R_{out}} + Q_{valve}(p_b(i), i)$$

**Method +1 in decubitus**

We can rewrite the full equation after the change of the setting of +1:

$$\frac{p_b(i) - p_{ss}}{R_{out}} + Q_{valve}(p_b(i), i) + Q_{ext} = \frac{p - p_{ss}}{R_{out}} + \frac{1}{E(p - p_0)}\frac{dp}{dt} + Q_{valve}(p, i + 1)$$

Therefore

$$\frac{dp}{dt} = E(p - p_0) \left[ \frac{p_b(i) - p}{R_{out}} + Q_{ext} + Q_{valve}(p_b(i), i) - Q_{valve}(p, i+1) \right]$$

The functioning point of de device jumps of + δQ from the curve i to i+1 after change of the selector setting of +1, the pressure being equal to $p_b(i)$ at t=0 according to the figure 11. There is then a relaxation up to an ICP equal to $p_b(i+1)$, the flow rate being approximated by:

$$Q_{valve}(p, i+1) = Q_{valve}(p_b(i), i+1)\frac{p}{p_b} = [Q_{valve}(p_b(i), i) + \delta Q]\frac{p}{p_b}$$

Finally the equation of the CSF dynamics state of the system satisfies to:

$$\frac{dp}{dt} = E(p - p_0) \left[ \frac{p_b(i) - p}{R_{out}} + Q_{ext} + Q_{valve}(p_b(i), i)\left(1 - \frac{p}{p_b}\right) - \delta Q \frac{p}{p_b} \right]$$

We assume now that $Q_{ext} = 0$.

This equation can be written in the form:

$$dt = \frac{dp}{\alpha + \beta p + \gamma p^2}$$

With

$$\alpha = -E p_0 \left( Q_{valve}(p_b(i), i) + \frac{p_b}{R_{out}} \right)$$

$$\beta = E \left( \frac{p_b + p_0}{R_{out}} + Q_{valve}(p_b(i), i)\left[1 + \frac{p_0}{p_b}\right] + \delta Q \frac{p_0}{p_b} \right)$$

$$\gamma = -E \left[ \frac{1}{R_{out}} + \frac{1}{p_b}(Q_{valve}(p_b(i), i) + \delta Q) \right]$$

General solutions of this equation are:

$$\frac{2\tan^{-1}\left(\frac{\beta + 2\gamma p}{\sqrt{4\alpha\gamma - \beta^2}}\right)}{\sqrt{4\alpha\gamma - \beta^2}}$$

Boundary conditions are:

$$p(t = 0) = p_b(i)$$

And

$$p(t = \infty) = p_b(i+1) = \left( \frac{Q_{valve}(p_b(i), i)}{Q_{valve}(p_b(i), i) + \delta Q} \right) p_b(i) = \frac{p_b(i)}{1 + \frac{\delta Q}{Q_{valve}(p_b(i), i)}}$$

The unknown parameters E, Rout and po can be determined by fitting the curve p(t) with the function tan$^{-1}$.

**Method +1 in vertical position**

The same test can be performed in vertical position, the analysis being simplified in the range of pressure $p_b(i)$ +$\Delta P_{reg}$ to $p_b(i)$ wherein the flow rate through the valve is constant:

$$\text{For } p_b(i) < p < p_b(i) + \Delta P_{reg}:$$

$$Q_{\text{valve}}(p + HP, i + 1) = Q_{\text{valve}}(p_b(i) + HP, i) + \delta Q$$

Where the hydrostatic pressure is noted HP.

We obtain

$$\frac{dp}{dt} = E(p - p_0)\left[\frac{p_b(i) - p}{R_{out}} + Q_{ext} + Q_{\text{valve}}(p_b(i) + HP, i) - Q_{\text{valve}}(p + HP, i + 1)\right]$$

Within this plateau of pressure, the equation for the CSF dynamics becomes, if we assume that $Q_{ext}$=0:

$$\frac{dp}{dt} = E(p - p_0)\left[\frac{p_b(i) - p}{R_{out}} - \delta Q\right]$$

We get:

$$dt = \frac{dp}{\alpha' + \beta'p + \gamma'p^2}$$

Leading to similar general solutions with the parameters:

$$\alpha' = -Ep_0\left(\frac{p_b}{R_{out}} - \delta Q\right)$$

$$\beta' = E\left(\frac{p_b + p_0}{R_{out}} - \delta Q\right)$$

$$\gamma' = -\frac{E}{R_{out}}$$

With the boundary condition

$$p(t = 0) = p_b(i)$$

**Method -1 in decubitus**

By changing the setting of the device of -1, we get:

$$\frac{dp}{dt} = E(p - p_0)\left[\frac{p_b(i) - p}{R_{out}} + Q_{ext} + Q_{valve}(p_b(i), i) - Q_{valve}(p, i - 1)\right]$$

We assume again that $Q_{ext} = 0$ and we restrict first the analysis on the plateau of the Q(p) curve, wherein the flow rate through the valve is constant. We assume also in a first approximation that $\Delta P_{reg}$ does not depends on i (see the figure 11).

The functioning point of de device jumps of - $\delta Q$ from the curve i to i-1 after change of the selector setting of -1, the pressure being equal to $p_b(i)$ at t=0 according to the figure 11.

For $p_b(i) < p < p_b(i) + \Delta P_{reg}$ the flow rate is constant:

$$Q_{valve}(p, i - 1) = Q_{valve}(p_b(i), i) - \delta Q$$

For $p_b(i) < p < p_b(i) + \Delta P_{reg}$ the equation of the CSF dynamics state of the system satisfies to:

$$\frac{dp}{dt} = E(p - p_0)\left[\frac{p_b(i) - p}{R_{out}} + \delta Q\right]$$

We get:

$$dt = \frac{dp}{\alpha'' + \beta'' p + \gamma'' p^2}$$

Leading to similar general solutions with the parameters:

$$\alpha'' = -Ep_0\left(\delta Q + \frac{p_b}{R_{out}}\right)$$

$$\beta'' = E\left(\frac{p_b + p_0}{R_{out}} + \delta Q\right)$$

$$\gamma'' = -\frac{E}{R_{out}}$$

With the boundary condition

$$p(t = 0) = p_b(i)$$

For $p > p_b(i) + \Delta P_{reg}$ the flow rate through the valve is:

$$Q_{valve}(p, i - 1) = p\left(\frac{Q_{valve}(p_b(i), i) - \delta Q}{p_b(i) + \Delta p_{reg}}\right)$$

This part of the curve could be analyzed using the same kind of general solutions, the boundary conditions including continuity conditions between the two ranges of pressure.

**Method -1 in vertical position**

The same test can be performed the patient being in vertical position, the same formula applies by changing $\Delta P_{reg}$ by HP, the hydrostatic pressure, those pressures being assumed to be equal in a first approximation.

In the latter case, we get:

$$\frac{dp}{dt} = E(p - p_0)\left[\frac{p_b(i) - p}{R_{out}} + Q_{ext} + Q_{valve}(p_b(i) + HP, i) - Q_{valve}(p + HP, i - 1)\right]$$

We assume that $Q_{ext} = 0$.

We can write, according to Figure 11, with $HP = \Delta p_{reg}$:

$$Q_{valve}(p + HP, i - 1) = \left(\frac{Q_{valve}(p_b(i) + HP, i) - \delta Q}{p_b(i) + HP}\right)(p + HP)$$

Therefore the differential equation for the CSF pressure is:

$$\frac{dp}{dt} = E(p - p_0)\left[\frac{p_b(i) - p}{R_{out}} + Q_{valve}(p_b(i) + HP, i)\left(1 - \frac{p + HP}{p_b(i) + HP}\right) - \delta Q\left(\frac{p + HP}{p_b(i) + HP}\right)\right]$$

We finally solve this equation using general formulae

$$dt = \frac{dp}{\alpha''' + \beta'''p + \gamma'''p^2}$$

Where

$$\alpha''' = -Ep_0\left(Q_{valve}(p_b(i) + HP, i) + \frac{p_b}{R_{out}} - \left(\frac{Q_{valve}(p_b(i) + HP, i) + \delta Q}{1 + \frac{p_b}{HP}}\right)\right)$$

$$\beta''' = E\left(\frac{p_b + p_0}{R_{out}} + Q_{valve}(p_b(i) + HP, i)\left[1 + \frac{p_0 - HP}{p_b + HP}\right] + \delta Q\left(\frac{p_0 - HP}{p_b + HP}\right)\right)$$

And

$$\gamma''' = -E\left[\frac{1}{R_{out}} + \frac{Q_{valve}(p_b(i) + HP, i) + \delta Q}{p_b + HP}\right]$$

In practice, a preliminary infusion test is performed to determine if a shunt shall be placed or not. During this test it is possible to determine these parameters E, Rout, po and even PP if a complete model is used (see annex).

The best strategy is therefore to use these parameters as constant and to check after implantation the good functioning of the device.

In functioning, it is also possible to determine the change of E and Rout with time in a non-invasive way by simply changing the setting of the device and by monitoring the pressure signal with time.

**Method summary:**

• Patient in decubitus

- Change the setting of the device of one unit
- Monitor the evolution of the pressure
- Analyse the pressure curve to determine the best fitting parameters E (brain elastance), Rout, Po and/or PP.
- Optionally, compare these values with preliminary data obtained before the shunt placement during an infusion test or just after the shunt implantation using this method
- Optionally, check if the potential change of pressure curve is due to change of the CSF outflow characteristics or a shunt failure

Thus the method for a non-invasive characterization of the CSF dynamics may comprise the following steps:

- Provide a medical device adapted to regulate a variable (such as a flow rate or a pressure) of the fluid which flows from the cranial cavity to another cavity of the body patient, the medical device comprising:

   ◦ An inlet located in the cranial cavity, in which the fluid enters in the medical device
   ◦ An outlet located in the other cavity, by which the fluid flows out the medical device
   ◦ An adjustable valve in fluid communication with the inlet and the outlet of the medical device, said adjustable valve being adapted to be set by a caregiver in such a way as to adjust the fluid variable,
   ◦ A pressure sensor which senses the differential pressure between the cranial cavity and the other cavity

- Change the fluid variable according to a new setting
- Monitor over time the differential pressure,
- Analyse the curve of the monitored differential pressure
- Determine E (brain elastance), Rout, P0 and/or PP (for example by using a mathematical model)

**Alternative method:**

- The parameters E, Rout, Po and PP are determined after implantation by an injection test via the filling of the reservoir septum with a needle and the monitoring of the pressure with time
- The change of setting test as described before is then performed to check the good functioning of the device by fitting the curve p(t) using the parameters obtained during the injection test

A general mathematical approach is given below:

We consider the full model that includes the peritoneum pressure PP. We only derive the equation for the method +1 in decubitus, other formula can be derived easily by applying the same formalism.

At rest, the equation of mass conservation writes:

$$Q_f = \frac{p_b(i) - p_{ss}}{R_{out}} + Q_{\text{valve}}(p_b(i) - PP, i)$$

We can rewrite the full equation after the change of the setting of +1:

$$\frac{p_b(i) - p_{ss}}{R_{out}} + Q_{\text{valve}}(p_b(i) - PP, i) + Q_{ext} = \frac{p - p_{ss}}{R_{out}} + \frac{1}{E(p - p_0)}\frac{dp}{dt} + Q_{\text{valve}}(p - PP, i + 1)$$

Therefore

$$\frac{dp}{dt} = E(p - p_0)\left[\frac{p_b(i) - p}{R_{out}} + Q_{ext} + Q_{\text{valve}}(p_b(i) - PP, i) - Q_{\text{valve}}(p - PP, i + 1)\right]$$

The functioning point of de device jumps of + δQ from the curve i to i+1 after change of the selector setting of +1, the pressure drop in the device being equal to $p_b(i)$-PP at t=0. There is then a relaxation up to an ICP equal to $p_b$(i+1), the flow rate being approximated by:

$$Q_{\text{valve}}(p - PP, i + 1) = Q_{\text{valve}}(p_b(i) - PP, i + 1)\left(\frac{p - PP}{p_b - PP}\right) = [Q_{\text{valve}}(p_b(i) - PP, i) + \delta Q]\left(\frac{p - PP}{p_b - PP}\right)$$

Finally the equation of the CSF dynamics state of the system satisfies to:

$$\frac{dp}{dt} = E(p - p_0)\left[\frac{p_b(i) - p}{R_{out}} + Q_{ext} + Q_{\text{valve}}(p_b(i) - PP, i)\left(1 - \frac{p - PP}{p_b - PP}\right) - \delta Q\left(\frac{p - PP}{p_b - PP}\right)\right]$$

We assume now that $Q_{ext} = 0$.

This equation can be written in the form:

$$dt = \frac{dp}{a + bp + cp^2}$$

With

$$a = -Ep_0\left(Q_{\text{valve}}(p_b - PP, i)\left(1 + \frac{1}{\frac{p_b}{PP} - 1}\right) + \frac{p_b}{R_{out}} + \delta Q\left(\frac{1}{\frac{p_b}{PP} - 1}\right)\right)$$

$$b = E\left(\frac{p_b + p_0}{R_{out}} + Q_{\text{valve}}(p_b(i) - PP, i)\left[1 + \frac{p_0 + PP}{p_b - PP}\right] + \delta Q\left(\frac{p_0 + PP}{p_b - PP}\right)\right)$$

$$c = -E\left[\frac{1}{R_{out}} + \frac{(Q_{\text{valve}}(p_b(i) - PP, i) + \delta Q)}{p_b - PP}\right]$$

General solutions of this equation are:

$$\frac{2\tan^{-1}\left(\frac{b + 2cp}{\sqrt{4ac - b^2}}\right)}{\sqrt{4ac - b^2}}$$

Boundary conditions are:

$$p(t = 0) = p_b(i)$$

And

$$p(t = \infty) = p_b(i + 1) = \frac{p_b(i) - PP}{1 + \frac{\delta Q}{Q_{\text{valve}}(p_b(i) - PP, i)}}$$

The unknown parameters E, Rout, PP and po can be determined by fitting the curve p(t) with the function $\tan^{-1}$.

**Claims**

1. A method for adjusting a hydrocephalus valve to a patient comprising the following steps:

- Providing a medical device adapted to regulate a fluid flow rate which flows from the cranial cavity to another cavity of the body patient, the medical device comprising:

  ◦ An inlet located in the cranial cavity, in which the fluid enters in the medical device
  ◦ An outlet located in the other cavity, by which the fluid flows out the medical device
  ◦ An adjustable valve in fluid communication with the inlet and the outlet of the medical device, said adjustable valve being adapted to be set by a user in such a way as to adjust the fluid flow rate of the medical device,
  ◦ A pressure sensor which senses the differential pressure between the cranial cavity and the other cavity

- Adjusting the fluid flow rate according to a first setting
- Monitoring over time the differential pressure,
- Analyzing the curve of the monitored differential pressure
- Adjusting the fluid flow rate according to a second setting while taking into account the differential pressure analysis

2. Method as in claim 1, wherein the curve analysis is the analysis of the curve slope.

3. Method as in claim 2, further comprising: determining the sign of the time derivative of the monitored differential pressure.

4. Method as in any previous claims, wherein the curve is compared to a reference curve.

5. Method as in the claim 4, wherein the reference curve reference is a curve obtained in a previous analysis.

6. Method as in any previous claims, wherein the adjusting step and the monitoring step are repeated until the curve is substantially planar.

7. Method as in any previous claims, wherein the patient is placed in a supine position during a predetermined period of time

8. Method as in any previous claims, wherein the patient is placed in a stand up position during a predetermined period of time

9. Method as in any previous claims further comprising the following step: changing the position of the patient from a supine position to a stand up position and/or from a stand up position to a supine position.

10. Method as in any previous claims, wherein the first setting is **characterized by** a first flow rate.

11. Method as in any previous claims, wherein the second setting is **characterized by** a second flow rate which is less than the first flow rate.

12. Method as in any previous claims further comprising: providing a remote controller comprising a screen which displays the data of the monitored differential pressure.

13. Method as in claim 12, wherein the medical device is adapted to communicate the differential pressure data to the remote controller in a wireless manner.

14. Method as in any previous claims, wherein the pressure sensor is arranged in the medical device.

15. Method for detecting a failure of a medical device comprising:

- Providing a medical device adapted to regulate a fluid which flows from the cranial cavity to another cavity of the body patient, the medical device comprising:

  ◦ An inlet located in the cranial cavity, in which the fluid enters in the medical device
  ◦ An outlet located in the other cavity, by which the fluid flows out the medical device
  ◦ A pressure sensor which senses the differential pressure between the cranial cavity and the other cavity

- Recording a first differential pressure measurement when the patient is in a first position,
- Changing the position of the patient
- Recording a second differential pressure measurement,
- Comparing the first and the second differential pressure data
- Determining a failure based on this comparison.

Pressure P$_{reservoir}$

Adjustable
gap

Adjustable force

FIG. 1

Pressure P$_{reservoir}$                     o-ring

Adjustable
gap

Adjustable force

FIG. 2

FIG. 3

FIG. 4

FIG. 5

FIG. 6

FIG. 7

FIG. 8

FIG. 9

FIG. 10

EP 3 192 554 A1

FIG. 11

FIG. 12

FIG. 13

FIG. 13 bis

FIG. 14

FIG. 15

41

FIG. 16

FIG. 17

FIG. 18

FIG. 19

FIG. 20

FIG. 21

Europäisches
Patentamt

European
Patent Office

Office européen
des brevets

# EUROPEAN SEARCH REPORT

Application Number

EP 16 15 1558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/196742 A1 (BROWD SAMUEL R [US] ET AL) 16 July 2015 (2015-07-16) * paragraphs [0007], [0026], [0031], [0074], [0078], [0081]; figures 1a,6a,7a,7d * | 1-14 | INV. A61M27/00 |
| X | EP 1 512 428 A1 (CODMAN & SHURTLEFF [US]) 9 March 2005 (2005-03-09) * paragraphs [0001], [0004], [0007], [0008], [0015]; figure 1 * | 1-14 | |
| X | US 2012/232461 A1 (SEAVER CHAD [US] ET AL) 13 September 2012 (2012-09-13) * paragraphs [0046], [0050] - [0052], [0055]; figures 1,3,5 * | 1-14 | |
| A | US 4 540 400 A (HOOVEN MICHAEL D [US]) 10 September 1985 (1985-09-10) * column 4, lines 4-23; figures 1,2 * * column 7, lines 66-68 * * column 8, lines 1-29 * * column 6, lines 13-32 * * column 1, lines 14-17 * | 1-14 | TECHNICAL FIELDS SEARCHED (IPC) A61M |

~~The present search report has been drawn up for all claims~~

| Place of search | Date of completion of the search | Examiner |
|---|---|---|
| Berlin | 28 July 2016 | Bielsa, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

Application Number

EP 16 15 1558

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 16 15 1558

The Search Division considers that the present European patent application does not comply with the requirements of unity of invention and relates to several inventions or groups of inventions, namely:

```
    1. claims: 1-14

        a method for adjusting a hydrocephalus valve to a patient.
                        ---

    2. claim: 15

        a method for detecting a failure of a medical device.
                        ---
```

EP 3 192 554 A1

## ANNEX TO THE EUROPEAN SEARCH REPORT
## ON EUROPEAN PATENT APPLICATION NO.

EP 16 15 1558

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2016

| Patent document cited in search report | Publication date | Patent family member(s) | Publication date |
|---|---|---|---|
| US 2015196742 A1 | 16-07-2015 | AU 2011227017 A1 | 11-10-2012 |
| | | CA 2793672 A1 | 22-09-2011 |
| | | CN 103002940 A | 27-03-2013 |
| | | EP 2547392 A1 | 23-01-2013 |
| | | US 2013197422 A1 | 01-08-2013 |
| | | US 2015196742 A1 | 16-07-2015 |
| | | WO 2011116393 A1 | 22-09-2011 |
| EP 1512428 A1 | 09-03-2005 | AT 390166 T | 15-04-2008 |
| | | AU 2004205267 A1 | 24-03-2005 |
| | | BR PI0403756 A | 07-06-2005 |
| | | CA 2480347 A1 | 05-03-2005 |
| | | DE 602004012676 T2 | 07-05-2009 |
| | | EP 1512428 A1 | 09-03-2005 |
| | | ES 2303625 T3 | 16-08-2008 |
| | | JP 5052745 B2 | 17-10-2012 |
| | | JP 2005131369 A | 26-05-2005 |
| | | US 2005055009 A1 | 10-03-2005 |
| US 2012232461 A1 | 13-09-2012 | AU 2012229290 A1 | 19-09-2013 |
| | | CA 2828427 A1 | 20-09-2012 |
| | | CN 103561799 A | 05-02-2014 |
| | | EP 2683426 A1 | 15-01-2014 |
| | | JP 2014514027 A | 19-06-2014 |
| | | US 2012232461 A1 | 13-09-2012 |
| | | WO 2012125449 A1 | 20-09-2012 |
| US 4540400 A | 10-09-1985 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 09709010 A
- EP 09807973 A
- EP 11710307 A
- EP 14706081 A
- EP 15175963 A

### Non-patent literature cited in the description

- **POLLAY M.** The function and structure of the cerebrospinal fluid outflow system. *Cerebrospinal Fluid Research,* 2010, vol. 7, 9
- **SOKOLOWSKI S.** Bolus injection test for measurement of cerebrospinal fluid absorption. *Journal of the Neurological Sciences,* 1976, vol. 28, 491-504
- **MARMAROU A. et al.** A nonlinear analysis of the cerebrospinal fluid system and intracranial pressure dynamics. *J. Neurosurg.,* 1978, vol. 48, 332-344
- **M. CLARKE et al.** The history of mathematical modeling in hydrocephalus. *Neurosurg. Focus,* 2007, vol. 22 (4), E3
- **SMILLIE A. et al.** A hydro-elastic model of hydrocephalus. *Journal of Fluid Mechanics,* 2005, vol. 539, 417-443
- **RAMAN K.** A stochastic differential equation analysis of cerebrospinal fluid dynamics. *Fluids Barriers CNS,* 2011, vol. 8, 9
- **CZOSNYKA M. et al.** Modeling of CSF dynamics: Legacy of Professor Anthony Marmarou. *Acta Neurochirurgica Supplementum,* 2012, vol. 113, 9
- **LEMAIRE J.J. et al.** Slow Pressure Waves in the Cranial Enclosure. *Acta Neurochir,* 2002, vol. 144, 243-254
- **MARMAROU et al.** Compartmental analysis of compliance and outflow resistance of the cerebrospinal fluid system. *J. Neurosurg.,* 1975, vol. 43, 523534
- **AVEZAAT C.J.J. et al.** Clinical observations on the relationship between cerebrospinal fluid pulse pressure and intracranial pressure. *Acta Neurochirurgica,* 1986, vol. 79, 13-29
- **CIESLICKI, K.** Mathematical modelling of the infusion test. *Pol. J. Med. Phys. Eng,* 2007, vol. 13, 33-54
- **EKLUND A. et al.** Assessment of cerebral fluid outflow resistance. *Med. Bio. Eng. Comput.,* 2007, vol. 45, 719-735
- **WEERAKKODY et al.** Clinical assessment of cerebrospinal fluid dynamics in hydrocephalus; Guide to interpretation based on observational study. *Acta Neurol. Scand.,* 2011, vol. 124, 85-98